# EUROPEAN PATENT SPECIFICATION

(11) **EP 1 021 535 B1**
(45) Date of publication and mention of the grant of the patent: **07.03.2007**
(21) Application number: 98949385.3
(22) Date of filing: 21.09.1998
(51) Int. Cl.: C12N 15/12, C07K 14/47, A61K 38/08, A61K 38/10, A61K 38/17, C12N 15/11, C12N 15/86, C07K 16/18, C12Q 1/68, A61K 35/14, A01K 67/027, C12N 5/08

(54) **HUMAN CANCER ANTIGEN NY ESO-1/CAG-3 AND GENE ENCODING SAME**
MENSCHLICHES KREBSANTIGEN NY ESO-1/CAG-3 UND DAZU KODIERENDES GEN
NOUVEL ANTIGENE DU CANCER HUMAIN NY ESO-1/CAG-3 ET GENE LE CODANT

(30) Priority: 08.10.1997 US 61428 P
(43) Date of publication of application: 26.07.2000
(73) Proprietor: THE GOVERNMENT OF THE UNITED STATES OF AMERICA, as represented by THE SECRETARY, DEPARTMENT OF HEALTH AND HUMAN SERVICES, Rockville, MD 20852 (US)
(72) Inventor: WANG, Rong, Fu, Bethesda, MD 20814 (US); ROSENBERG, Steven, A., Potomac, MD 20854 (US)
(74) Representative: Schnappauf, Georg
(86) International application number: PCT/US1998/019609
(87) International publication number: WO 1999/018206

(56) References cited:
- WO-A-97/29195
- WO-A-98/14464
- WO-A-98/32855
- CHEN Y -T ET AL: "A TESTICULAR ANTIGEN ABERRANTLY EXPRESSED IN HUMAN CANCERS DETECTED BY AUTOLOGOUS ANTIBODY SCREENING" PROCEEDINGS OF THE NATIONAL ACADEMY OF SCIENCES OF USA, vol. 94, March 1997, pages 1914-1918, XP002064909
- VAN ELSAS, A. ET AL.: "Transformation of IL-2 augments CTL response to human melanoma cells in vitro: immunological characterization of a melanoma vaccine." JOURNAL OF IMMUNOTHERAPY, vol. 20, no. 5, September 1997, pages 343-53, XP002096030
- PARKHURST, M.R. ET AL.: "Improved induction of melanoma-reactive CTL with peptides from the melanoma antigen gp100 modified at HLA-A*0201-binding residues." JOURNAL OF IMMUNOLOGY, vol. 157, 1996, pages 2539-48, XP002096010
- JÄGER, E. ET AL.: "Simultaneous humoral and cellular immune response against cancer-testis antigen NY-ESO-1: definition of human histocompatibility leukocyte antigen (HLA)-A2-binding peptide epitopes." JOURNAL OF EXPERIMENTAL MEDICIN, vol. 187, no. 2, 19 January 1998, pages 265-70, XP002096011

### SEQUENCE LISTING

<110> Government of the United States of America, represented by the Secretary, Department of Health and Human Services
<120> NOVEL HUMAN CANCER ANTIGEN NY ESO-1/CAG-3 AND GENE ENCODING SAME
<130> 218802
<140> 98949385.3
   <141> 2000-05-05
<150> PCT/US98/19609
   <151> 1998-09-21
<150> 60/061,428
   <151> 1997-10-08
<160> 106
<170> PatentIn version 3.2
<210> 1
   <211> 805
   <212> DNA
   <213> Homo sapiens
<400> 1
   agcagggggc gctgtgtgta ccgagaatac gagaatacct cgtgggccct gaccttctct      60
   ctgagagccg ggcagaggct ccggagccat gcaggccgaa ggccggggca cagggggttc    120
   gacgggcgat gctgatggcc caggaggccc tggcattcct gatggcccag ggggcaatgc      180
   tggcggccca ggagaggcgg gtgccacggg cggcagaggt ccccggggcg caggggcagc      240
   aagggcctcg gggccgggag gaggcgcccc gcggggtccg catggcggcg cggcttcagg     300
   gctgaatgga tgctgcagat gcggggccag ggggccggag agccgcctgc ttgagttcta      360
   cctcgccatg cctttcgcga cacccatgga agcagagctg gcccgcagga gcctggccca     420
   ggatgcccca ccgcttcccg tgccaggggt gcttctgaag gagttcactg tgtccggcaa     480
   catactgact atccgactga ctgctgcaga ccaccgccaa ctgcagctct ccatcagctc     540
   ctgtctccag cagctttccc tgttgatgtg gatcacgcag tgctttctgc ccgtgttttt     600
   ggctcagcct ccctcagggc agaggcgcta agcccagcct ggcgcccctt cctaggtcat     660
   gcctcctccc ctagggaatg gtcccagcac gagtggccag ttcattgtgg gggcctgatt     720
   gtttgtcgct ggaggaggac ggcttacatg tttgtttctg tagaaaataa aactgagcta     780
   cgaaaaaaaa aaaaaaaaaa aaaaa                                           805
<210> 2
   <211> 540
   <212> DNA
   <213> Homo sapiens
<400> 2
   atgcaggccg aaggccgggg cacagggggt tcgacgggcg atgctgatgg cccaggaggc 60
   cctggcattc ctgatggccc agggggcaat gctggcggcc caggagaggc gggtgccacg 120
   ggcggcagag gtccccgggg cgcaggggca gcaagggcct cggggccggg aggaggcgcc 180
   ccgcggggtc cgcatggcgg cgcggcttca gggctgaatg gatgctgcag atgcggggcc 240
   agggggccgg agagccgcct gcttgagttc tacctcgcca tgcctttcgc gacacccatg 300
   gaagcagagc tggcccgcag gagcctggcc caggatgccc caccgcttcc cgtgccaggg 360
   gtgcttctga aggagttcac tgtgtccggc aacatactga ctatccgact gactgctgca 420
   gaccaccgcc aactgcagct ctccatcagc tcctgtctcc agcagctttc cctgttgatg 480
   tggatcacgc agtgctttct gcccgtgttt ttggctcagc ctccctcagg gcagaggcgc 540
<210> 3
   <211> 174
   <212> DNA
   <213> Homo sapiens
<400> 3
   atgctgatgg cccaggaggc cctggcattc ctgatggccc agggggcaat gctggcggcc 60
   caggagaggc gggtgccacg ggcggcagag gtccccgggg cgcaggggca gcaagggcct 120
   cggggccggg aggaggcgcc ccgcggggtc cgcatggcgg cgcggcttca gggc 174
<210> 4
   <211> 180
   <212> PRT
   <213> Homo sapiens
<400> 4
   Met Gln Ala Glu Gly Arg Gly Thr Gly Gly Ser Thr Gly Asp Ala Asp
   1 5 10 15
   Gly Pro Gly Gly Pro Gly Ile Pro Asp Gly Pro Gly Gly Asn Ala Gly
   20 25 30
   Gly Pro Gly Glu Ala Gly Ala Thr Gly Gly Arg Gly Pro Arg Gly Ala
   35 40 45
   Gly Ala Ala Arg Ala Ser Gly Pro Gly Gly Gly Ala Pro Arg Gly Pro
   50 55 60
   His Gly Gly Ala Ala Ser Gly Leu Asn Gly Cys Cys Arg Cys Gly Ala
   65 70 75 80
   Arg Gly Pro Glu Ser Arg Leu Leu Glu Phe Tyr Leu Ala Met Pro Phe
   85 90 95
   Ala Thr Pro Met Glu Ala Glu Leu Ala Arg Arg Ser Leu Ala Gln Asp
   100 105 110
   Ala Pro Pro Leu Pro Val Pro Gly Val Leu Leu Lys Glu Phe Thr Val
   115 120 125
   Ser Gly Asn Ile Leu Thr Ile Arg Leu Thr Ala Ala Asp His Arg Gln
   130 135 140
   Leu Gln Leu Ser Ile Ser Ser Cys Leu Gln Gln Leu Ser Leu Leu Met
   145 150 155 160
   Trp Ile Thr Gln Cys Phe Leu Pro Val Phe Leu Ala Gln Pro Pro Ser
   165 170 175
   Gly Gln Arg Arg
   180
<210> 5
   <211> 58
   <212> PRT
   <213> Homo sapiens
<400> 5
   Met Leu Met Ala Gln Glu Ala Leu Ala Phe Leu Met Ala Gln Gly Ala
   1 5 10 15
   Met Leu Ala Ala Gln Glu Arg Arg Val Pro Arg Ala Ala Glu Val Pro
   20 25 30
   Gly Ala Gln Gly Gln Gln Gly Pro Arg Gly Arg Glu Glu Ala Pro Arg
   35 40 45
   Gly Val Arg Met Ala Ala Arg Leu Gln Gly
   50 55
<210> 6
   <211> 9
   <212> PRT
   <213> Homo sapiens
<400> 6
   Ala Gln Pro Pro Ser Gly Gln Arg Arg
   1 5
<210> 7
   <211> 9
   <212> PRT
   <213> Homo sapiens
<400> 7
   Thr Pro Met Glu Ala Glu Leu Ala Arg
   1 5
<210> 8
   <211> 9
   <212> PRT
   <213> Homo sapiens
<400> 8
   Pro Met Glu Ala Glu Leu Ala Arg Arg
   1 5
<210> 9
   <211> 9
   <212> PRT
   <213> Homo sapiens
<400> 9
   Gly Ala Thr Gly Gly Arg Gly Pro Arg
   1 5
<210> 10
   <211>. 9
   <212> PRT
   <213> Homo sapiens
<400> 10
<210> 11
   <211> 9
   <212> PRT
   <213> Homo sapiens
<400> 11
   Leu Ala Gln Pro Pro Ser Gly Gln Arg
   1 5
<210> 12
   <211> 9
   <212> PRT
   <213> Homo sapiens
<400> 12
   Val Ser Gly Asn Ile Leu Thr Ile Arg
   1 5
<210> 13
   <211> 9
   <212> PRT
   <213> Homo sapiens
<400> 13
   Ile Arg Leu Thr Ala Ala Asp His Arg
   1 5
<210> 14
   <211> 9
   <212> PRT
   <213> Homo sapiens
<400> 14
   Ser Gly Pro Gly Gly Gly Ala Pro Arg
   1 5
<210> 15
   <211> 10
   <212> PRT
   <213> Homo sapiens
<400> 15
   Thr Val Ser Gly Asn Ile Leu Thr Ile Arg
   1 5 10
<210> 16
   <211> 10
   <212> PRT
   <213> Homo sapiens
<400> 16
   Thr Ile Arg Leu Thr Ala Ala Asp His Arg
   1 5 10
<210> 17
   <211> 10
   <212> PRT
   <213> Homo sapiens
<400> 17
   Ala Thr Pro Met Glu Ala Glu Leu Ala Arg
   1 5 10
<210> 18
   <211> 10
   <212> PRT
   <213> Homo sapiens
<400> 18
   Phe Leu Ala Gln Pro Pro Ser Gly Gln Arg
   1 5 10
<210> 19
   <211> 10
   <212> PRT
   <213> Homo sapiens
<400> 19
   Thr Pro Met Glu Ala Glu Leu Ala Arg Arg
   1               5                   10
<210> 20
   <211> 10
   <212> PRT
   <213> Homo sapiens
<400> 20
   Arg Cys Gly Ala Arg Gly Pro Glu Ser Arg
   1               5                   10
<210> 21
   <211> 10
   <212> PRT
   <213> Homo sapiens
<400> 21
   Ala Ala Ser Gly Leu Asn Gly Cys Cys Arg
   1               5                   10
<210> 22
   <211> 10
   <212> PRT
   <213> Homo sapiens
<400> 22
   Leu Ala Gln Pro Pro Ser Gly Gln Arg Arg
   1               5                   10
<210> 23
   <211> 10
   <212> PRT
   <213> Homo sapiens
<400> 23
   Arg Gly Pro Arg Gly Ala Gly Ala Ala Arg
   1               5                   10
<210> 24
   <211> 10
   <212> PRT
   <213> Homo sapiens
<400> 24
   Leu Asn Gly Cys Cys Arg Cys Gly Ala Arg
   1               5                   10
<210> 25
   <211> 10
   <212> PRT
   <213> Homo sapiens
<400> 25
   Ala Ser Gly Pro Gly Gly Gly Ala Pro Arg
   1               5                   10
<210> 26
   <211> 15
   <212> PRT
   <213> Homo sapiens
<400> 26
   Ala Gly Ala Ala Arg Ala Ser Gly Pro Gly Gly Gly Ala Pro Arg
   1               5                   10                  15
<210> 27
   <211> 14
   <212> PRT
   <213> Homo sapiens
<400> 27
   Gly Ala Ala Arg Ala Ser Gly Pro Gly Gly Gly Ala Pro Arg
   1               5                   10
<210> 28
   <211> 13
   <212> PRT
   <213> Homo sapiens
<400> 28
   Ala Ala Arg Ala Ser Gly Pro Gly Gly Gly Ala Pro Arg
   1               5                   10
<210> 29
   <211> 12
   <212> PRT
   <213> Homo sapiens
<400> 29
   Ala Arg Ala Ser Gly Pro Gly Gly Gly Ala Pro Arg
   1 5 10
<210> 30
   <211> 11
   <212> PRT
   <213> Homo sapiens
<400> 30
   Arg Ala Ser Gly Pro Gly Gly Gly Ala Pro Arg
   1 5 10
<210> 31
   <211> 8
   <212> PRT
   <213> Homo sapiens
<400> 31
   Gly Pro Gly Gly Gly Ala Pro Arg
   1 5
<210> 32
   <211> 11
   <212> PRT
   <213> Homo sapiens
<400> 32
   Ala Ser Gly Pro Gly Gly Gly Ala Pro Arg Gly
   1 5 10
<210> 33
   <211> 10
   <212> PRT
   <213> Homo sapiens
<400> 33
   Ser Gly Pro Gly Gly Gly Ala Pro Arg Gly
   1 5 10
<210> 34
   <211> 10
   <212> PRT
   <213> Homo sapiens
<400> 34
   Ala Ala Gly Pro Gly Gly Gly Ala Pro Arg
   1 5 10
<210> 35
   <211> 10
   <212> PRT
   <213> Homo sapiens
<400> 35
   Ala Ile Gly Pro Gly Gly Gly Ala Pro Arg
   1 5 10
<210> 36
   <211> 10
   <212> PRT
   <213> Homo sapiens
<400> 36
   Ala Leu Gly Pro Gly Gly Gly Ala Pro Arg
   1 5 10
<210> 37
   <211> 10
   <212> PRT
   <213> Homo sapiens
<400> 37
   Ala Val Gly Pro Gly Gly Gly Ala Pro Arg
   1 5 10
<210> 38
   <211> 10
   <212> PRT
   <213> Homo sapiens
<400> 38
   Ala Thr Gly Pro Gly Gly Gly Ala Pro Arg
   1 5 10
<210> 39
   <211> 10
   <212> PRT
   <213> Homo sapiens
<400> 39
   Ala Ser Gly Pro Gly Gly Gly Ala Pro Lys
   1 5 10
<210> 40
   <211> 10
   <212> PRT
   <213> Homo sapiens
<400> 40
   Ala Ser Gly Pro Gly Gly Gly Ala Pro His
   1 5 10
<210> 41
   <211> 1Q
   <212> PRT
   <213> Homo sapiens
<400> 41
<210> 42
   <211> 10
   <212> PRT
   <213> Homo sapiens
<400> 42
   Val Ser Gly Pro Gly Gly Gly Ala Pro Arg
   1 5 10
<210> 43
   <211> 10
   <212> PRT
   <213> Homo sapiens
<400> 43
   Leu Ser Gly Pro Gly Gly Gly Ala Pro Arg
   1 5 10
<210> 44
   <211> 10
   <212> PRT
   <213> Homo sapiens
<400> 44
   Arg Ser Gly Pro Gly Gly Gly Ala Pro Arg
   1 5 10
<210> 45
   <211> 20
   <212> PRT
   <213> Homo sapiens
<400> 45
   Arg Gly Pro Arg Gly Ala Gly Ala Ala Arg Ala Ser Gly Pro Gly Gly
   1 5 10 15
   Gly Ala Pro Arg
   20
<210> 46
   <211> 9
   <212> PRT
   <213> Homo sapiens
<400> 46.
   Ala Ala Gln Glu Arg Arg Val Pro Arg
   1 5
<210> 47
   <211> 10
   <212> PRT
   <213> Homo sapiens
<400> 47
   Leu Ala Ala Gln Glu Arg Arg Val Pro Arg
   1 5 10
<210> 48
   <211> 11
   <212> PRT
   <213> Homo sapiens
<400> 48
   Met Leu Ala Ala Gln Glu Arg Arg Val Pro Arg
   1 5 10
<210> 49
   <211> 12
   <212> PRT
   <213> Homo sapiens
<400> 49
   Ala Met Leu Ala Ala Gln Glu Arg Arg Val Pro Arg
   1 5 10
<210> 50
   <211> 13
   <212> PRT
   <213> Homo sapiens
<400> 50
   Gly Ala Met Leu Ala Ala Gln Glu Arg Arg Val Pro Arg
   1 5 10
<210> 51
   <211> 30
   <212> DNA
   <213> Homo sapiens
<400> 51
   cctcggggcc gggaggaggc gccccgcggg 30
<210> 52
   <211> 30
   <212> DNA
   <213> Homo sapiens
<400> 52
   ctggcggccc aggagaggcg ggtgccacgg 30
<210> 53
   <211> 27
   <212> DNA
   <213> Homo sapiens
<400> 53
   gcggcccagg agaggcgggt gccacgg 27
<210> 54
   <211> 11
   <212> PRT
   <213> Homo sapiens
<220>
   <221> misc_feature
   <222> (1)..(1)
   <223> xaa is no amino acid or one to about 10 amino acids
<220>
   <221> misc_feature
   <222> (2)..(2)
   <223> xaa is Ala, Thr, val, Leu or Arg
<220>
   <221> misc feature
   <222> (3)..(3)
   <223> xaa is Ser or conservative substitution
<220>
   <221> misc_feature
   <222> (11)..(11)
   <223> xaa is Arg or Lys
<400> 54
   Xaa Xaa Xaa Gly Pro Gly Gly Gly Ala Pro Xaa
   1 5 10
<210> 55
   <211> 61
   <212> PRT
   <213> Homo sapiens
<400> 55
   Arg Cys Gly Ala Arg Gly Pro Glu Ser Arg Leu Leu Glu Phe Tyr Leu
   1 5 10 15
   Ala Met Pro Phe Ala Thr Pro Met Glu Ala Glu Leu Ala Arg Arg Ser
   20 25 30
   Leu Ala Gln Asp Ala Pro Pro Leu Pro Val Pro Gly Val Leu Leu Lys
   35 40 45
   Glu Phe Thr Val Ser Gly Asn Ile Leu Thr Ile Arg Leu
   50 55 60
<210> 56
   <211> 25
   <212> PRT
   <213> Homo sapiens
<400> 56
   Met Leu Met Ala Gln Glu Ala Leu Ala Phe Leu Met Ala Gln Gly Ala
   1 5 10 15
   Met Leu Ala Ala Gln Glu Arg Arg Val
   20 25
<210> 57
   <211> 15
   <212> PRT
   <213> Homo sapiens
<400> 57
   Ala Gly Arg Leu Tyr Leu Pro Leu Pro Pro Val Pro Val Leu Leu
   1 5 10 15
<210> 58
   <211> 24 .
   <212> PRT
   <213> Homo sapiens
<400> 58
   Gly Gly Pro Leu Leu Glu Phe Leu Met Pro Thr Leu Ala Arg Ser Leu
   1 5 10 15
   Ala Ala Leu Pro Leu Glu Gly Leu
   20
<210> 59
   <211> 11
   <212> PRT
   <213> Homo sapiens
<400> 59
   Met Glu Ala Leu Phe Leu Met Gln Met Ala Val
   1 5 10
<210> 60
   <211> 48
   <212> PRT
   <213> Homo sapiens
<400> 60
   Gln Ala Ala Ala Thr Gly Gly Asp Ala Arg Gln Leu Val Gly Tyr Leu
   1 5 10 15
   Val Ser Gln Ser Gly Leu Pro Leu Asp Thr Ser Ala Leu Gln Ala Gln
   20 25 30
   Leu Arg Glu Thr Leu Pro Pro His Met Val Pro Val Val Leu Leu Gln
   35 40 45
<210> 61
   <211> 64
   <212> PRT
   <213> Homo sapiens
<400> 61
   Gln Ala Gly Val Ala Gly Pro Ala Ala Ala Leu Leu Glu Phe Thr Leu
   1               5                   10                  15
   Asn Met Leu Pro Trp Lys Thr Ala Val Gly Asp Phe Leu Ala Ser Thr
               20                  25                  30
   Arg Leu Ser Leu Ala Asp Val Ala Ala His Leu Pro Leu Val Gln His
           35                  40                  45
   Val Leu Asp Glu Asn Ser Leu Ile Gly Arg Leu Ala Leu Ala Lys Leu
       50                  55                  60
<210> 62
   <211> 25
   <212> PRT
   <213> Homo sapiens
<400> 62
   Met Pro Thr Thr Asn Glu Ala Leu Arg Phe Leu Met Gln Gln Pro Asn
   1               5                   10                  15
   Met Val Val Ala Pro Ser Lys Ala Val
               20                  25
<210> 63
   <211> 9
   <212> PRT.
   <213> Homo sapiens
<400> 63
   Arg Leu Leu Glu Phe Tyr Leu Ala Met
   1               5
<210> 64
   <211> 9
   <212> PRT
   <213> Homo sapiens
<400> 64
   Gln Gln Leu Ser Leu Leu Met Trp Ile
   1               5
<210> 65
   <211> 9
   <212> PRT
   <213> Homo sapiens
<400> 65
   Leu Pro Val Pro Gly Val Leu Leu Lys
   1 5
<210> 66
   <211> 9
   <212> PRT
   <213> Homo sapiens
<400> 66
   Gly Val Leu Leu Lys Glu Phe Thr Val
   1 5
<210> 67
   <211> 9
   <212> PRT
   <213> Homo sapiens
<400> 67
   Asn Ile Leu Thr Ile Arg Leu Thr Ala
   1 5
<210> 68
   <211> 9
   <212> PRT
   <213> Homo sapiens
<400> 68
   Trp Ile Thr Gln Cys Phe Leu Pro Val
   1 5
<210> 69
   <211> 9
   <212> PRT
   <213> Homo sapiens
<400> 69
   Thr Val Ser Gly Asn Ile Leu Thr Ile
   1 5
<210> 70
   <211> 9
   <212> PRT
   <213> Homo sapiens
<400> 70
   Leu Gln Gln Leu Ser Leu Leu Met Trp
   1               5
<210> 71
   <211> 9
   <212> PRT
   <213> Homo sapiens
<400> 71
   Leu Met Trp Ile Thr Gln Cys Phe Leu
   1               5
<210> 72
   <211> 9
   <212> PRT
   <213> Homo sapiens
<400> 72
   Leu Leu Met Trp Ile Thr Gln Cys Phe
   1               5
-<210> 73
   <211> 9
   <212> PRT
   <213> Homo sapiens
<400> 73
<210> 74
   <211> 9
   <212> PRT
   <213> Homo sapiens
<400> 74
   Ser Ile Ser Ser Cys Leu Gln Gln Leu
   1               5
<210> 75
   <211> 9
   <212> PRT
   <213> Homo sapiens
<400> 75
   Leu Gln Leu Ser Ile Ser Ser Cys Leu
   1 5
<210> 76
   <211> 9
   <212> PRT
   <213> Homo sapiens
<400> 76
   Cys Leu Gln Gln Leu Ser Leu Leu Met
   1 5
<210> 77
   <211> 9
   <212> PRT
   <213> Homo sapiens
<400> 77
   Ala Gln Asp Ala Pro Pro Leu Pro Val
   1 5
<210> 78
   <211> 9
   <212> PRT
   <213> Homo sapiens
<400> 78
   Gln Cys Phe Leu Pro Val Phe Leu Ala
   1 5
<210> 79
   <211> 9
   <212> PRT
   <213> Homo sapiens
<400> 79
   Arg Gln Leu Gln Leu Ser Ile Ser Ser
   1 5
<210> 80
   <211> 9
   <212> PRT
   <213> Homo sapiens
<400> 80
   Ser Leu Ala Gln Asp Ala Pro Pro Leu
   1 5
<210> 81
   <211> 9
   <212> PRT
   <213> Homo sapiens
<400> 81
   Asn Gly Cys Cys Arg Cys Gly Ala Arg
   1 5
<210> 82
   <211> 9
   <212> PRT
   <213> Homo sapiens
<400> 82
   Thr Ile Arg Leu Thr Ala Ala Asp His
   1 5
<210> 83
   <211> 9
   <212> PRT
   <213> Homo sapiens
<400> 83
   Ala Ser Gly Leu Asn Gly Cys Cys Arg
   1 5
<210> 84
   <211> 10
   <212> PRT
   <213> Homo sapiens
<400> 84
   Thr Val Ser Gly Asn Ile Leu Thr Ile Arg
   1 5 10
<210> 85
   <211> 10
   <212> PRT
   <213> Homo sapiens
<400> 85
   Thr Ile Arg Leu Thr Ala Ala Asp His Arg
   1 5 10
<210> 86
   <211> 10
   <212> PRT
   <213> Homo sapiens
<400> 86
   Thr Gln Cys Phe Leu Pro Val Phe Leu Ala
   1 5 10
<210> 87
   <211> 10
   <212> PRT
   <213> Homo sapiens
<400> 87
   Leu Gln Gln Leu Ser Leu Leu Met Trp Ile
   1 5 10
<210> 88
   <211> 10
   <212> PRT
   <213> Homo sapiens
<400> 88
   Pro Leu Pro Val Pro Gly Val Leu Leu Lys
   1 5 10
<210> 89
   <211> 10
   <212> PRT
   <213> Homo sapiens
<400> 89
   Cys Leu Gln Gln Leu Ser Leu Leu Met Trp
   1 5 10
<210> 90
   <211> 10
   <212> PRT
   <213> Homo sapiens
<400> 90
   Asn Ile Leu Thr Ile Arg Leu Thr Ala Ala
   1               5                   10
<210> 91
   <211> 10
   <212> PRT
   <213> Homo sapiens
<400> 91
   Phe Thr Val Ser Gly Asn Ile Leu Thr Ile
   1               5                   10
<210> 92
   <211> 10
   <212> PRT
   <213> Homo sapiens
<400> 92
   Leu Leu Met Trp Ile Thr Gln Cys Phe Leu
   1               5                   10
<210> 93
   <211> 10
   <212> PRT
   <213> Homo sapiens
<400> 93
   Leu Leu Glu Phe Tyr Leu Ala Met Pro Phe
   1               5                   10
<210> 94
   <211> 10
   <212> PRT
   <213> Homo sapiens
<400> 94
   Trp Ile Thr Gln Cys Phe Leu Pro Val Phe
   1               5                   10
<210> 95
   <211> 10
   <212> PRT
   <213> Homo sapiens
<400> 95
   Ser Leu Leu Met Trp Ile Thr Gln Cys Phe
   1 5 10
<210> 96
   <211> 10
   <212> PRT
   <213> Homo sapiens
<400> 96
   Ala Met Pro Phe Ala Thr Pro Met Glu Ala
   1 5 10
<210> 97
   <211> 10
   <212> PRT
   <213> Homo sapiens
<400> 97
   Gln Gln Leu Ser Leu Leu Met Trp Ile Thr
   1 5 10
<210> 98
   <211> 10
   <212> PRT
   <213> Homo sapiens
<400> 98
   Arg Leu Leu Glu Phe Tyr Leu Ala Met Pro
   1 5 10
<210> 99
   <211> 10
   <212> PRT
   <213> Homo sapiens
<400> 99
   Arg Gln Leu Gln Leu Ser Ile Ser Ser Cys
   1 5 10
<210> 100
   <211> 9
   <212> PRT
   <213> Homo sapiens
<400> 100
   Gly Leu Gly Cys Cys Arg Cys Gly Ala
   1 5
<210> 101
   <211> 10
   <212> PRT
   <213> Homo sapiens
<400> 101
   Tyr Leu Ala Met Pro Phe Ala Thr Pro Met
   1 5 10
<210> 102
   <211> 10
   <212> PRT
   <213> Homo sapiens
<400> 102
   Gly Ile Pro Asp Gly Pro Gly Gly Asn Ala
   1 5 10
<210> 103
   <211> 10
   <212> PRT
   <213> Homo sapiens
<400> 103
   Gln Leu Gln Leu Ser Ile Ser Ser Cys Leu
   1 5 10

## Description

### Field of the Invention

The present invention relates to the area of cancer diagnostics and therapeutics including a cancer vaccine. More specifically, the invention relates to a novel human cancer peptide or a functional portion or derivative thereof, derived from an alternative open reading frame of the NY ESO-1/CAG-3 gene, and to an isolated nucleic acid encoding the cancer peptide.

### Background of the Invention

The adoptive transfer of tumor infiltrating lymphocytes (TIL) can mediate tumor regression in patients with metastatic melanoma, suggesting that tumor rejection antigens recognized by T cells exist on these tumor cells. The availability of such T cells has made it possible to clone and sequence the genes that encode human melanoma antigens. The antigens identified so far from human melanoma can be divided into two classes based on their expression pattern. The antigens of the first class are encoded by genes that are expressed only in tumor and testis, but not other normal human tissues. MAGE1, MAGE3, GAGE and BAGE are examples of this class. The second class of antigens represents differentiation antigens encoded by genes that are expressed only in melanocytes, melanomas, and normal retina. MART-1/Melan-A, gp100 and tyrosine are examples of this class. All these antigens are nonmutated self proteins. However, several mutated antigens were also identified to be recognized by T cells, including CDK 4, B-catenin and Mum-1. Identification of the antigenic epitopes recognized by T cells derived from the corresponding gene products is important not only for understanding the mechanism of immune response to self antigens, but also for developing new, effective immunotherapeutic strategies with these antigens or synthetic peptides for the treatment of patients with cancer.

Previous studies showed that the infusion of TIL586 plus IL-2 into an autologous patient with melanoma resulted in the objective regression ofmetastases. More recently, the gene, tyrosinase-related protein 1 (TRP-1 or gp75) was cloned which encodes the tumor antigen recognized by TIL586 in association with HLA-A31. Interestingly, the gene product, gp75, was originally identified as an antigen recognized by IgG antibodies in the serum from a patient with metastatic melanoma. The gene was found to be expressed only in melanoma, normal melanocyte cell lines, and retina, but not in other normal tissues tested. Therefore, this gene is a member of the second class of antigens including MART-1/Melan-A, gp100 and tyrosinase.

Wang, R. F. et al.J. Exp. Med. Vol. 183, pp. 1131-1140 (1996) report on the identification of a cancer peptide encoded from an alternative open reading frame sequence within the TRP-1 gene which is specifically recognized by cloned TIL 586 cells. Wang, R.F. et al J. Exp. Med. Vol 184, pp. 2207-2216 (1996) identified a second tumor antigen, TRP-2, recognized by a HLA-A31-restricted CTL clone derived from the TIL 586 cell line.

The present invention is the identification and isolation of novel tumor antigens distinct from TRP-1 and TRP-2 which are recognized by CTL clones derived from the TIL 586 cell line. The cancer peptides of the invention are useful as an immunogen and vaccine to inhibit or prevent cancer in a mammal and as a diagnostic agent to detect cancer or precancer.

### Summary of the Invention

One object of the present invention is to provide a novel peptide and portions thereof recognized as a cancer antigen by T lymphocytes.

The cancer peptides of the present invention are encoded by all or a portion of an alternative open reading frame (SEQ ID NO:3) of the NY ESO-1/CAG-3 (term used interchangeably herein with CAG-3) gene (SEQ ID NO:1). CAG-3 is a potent tumor antigen capable of eliciting an antigen specific immune response by T cells.

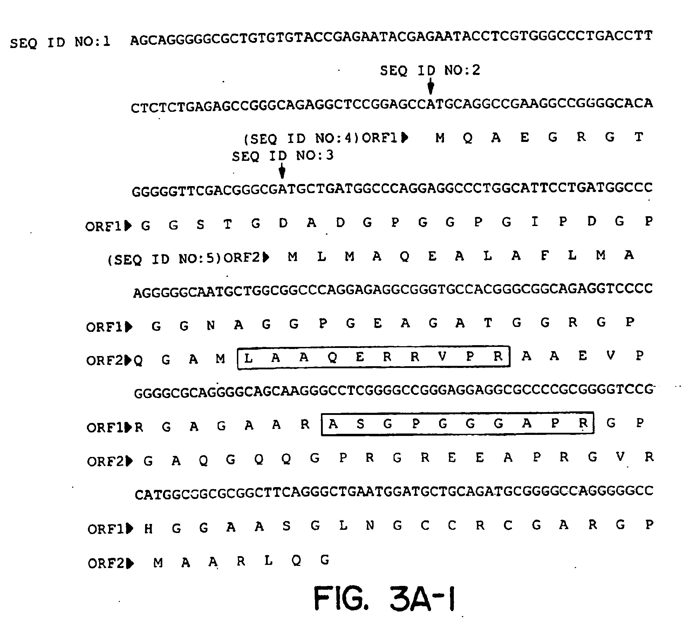

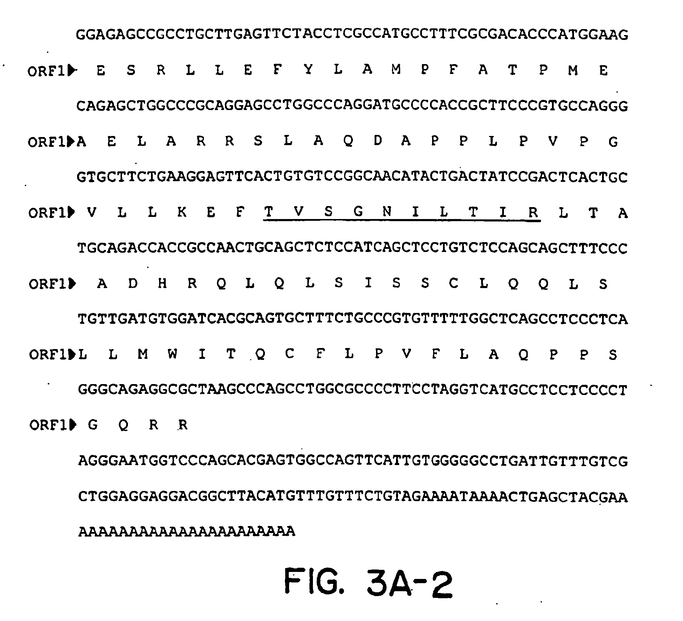

One aspect of the invention is an isolated cancer peptide comprising the amino acid sequence MLMAQEALAF LMAQGAMLAA QERRVPRAAE VPGAQGQQGP RGREEAPRGV RMAARLQG (SEQ ID NO: 5), or a functional portion or derivative thereof, wherein said functional portion or derivative is immunologically recognized by antigen specific cytotoxic T lymphocytes specific for the peptide having the sequence of SEQ ID NO: 5 of Figure 3A.

Another aspect of the present invention is an isolated cancer peptide, portion or derivative thereof, encoded by wherein the portion or derivative thereof is recognized by antigen specific cytotoxic T cells specific for the peptide having the sequence of SEQ ID NO: 5 of Figure 3A.

Another aspect of the present invention is a pharmaceutical composition comprising at least one cancer peptide of the invention and a pharmaceutically acceptable carrier. Another aspect of the present invention is an immunogen comprising the cancer peptide of the invention alone or in combination with at least one immunostimulatory molecule, wherein said immunogen elicits antigen specific cytotoxic T-lymphocytes. The pharmaceutical composition is useful in treating or preventing cancer in a mammal wherein the pharmaceutical composition is administered to the mammal in an amount effective in preventing or inhibiting the cancer in the mammal.

A further aspect of the invention is an isolated nucleic acid encoding a cancer peptide of the invention, wherein the nucleic acid does not encode SEQ ID NO:4 of Figure 3A. The invention further provides oligonucleotides consisting of a nucleic acid complementary to the portion of the nucleic acid sequence of SEQ ID NO:3 encoding the peptide having the sequence of SEQ ID NO:5 for use as probes or primers.

The present invention further provides a recombinant expression vector comprising the above-mentioned nucleic acid encoding a cancer peptide of the invention alone or in combination with a DNA sequence encoding at least one immunostimulatory molecule.

The present invention also provides a recombinant virus having incorporated into the viral genome or portion thereof the above-mentioned nucleic acid encoding a cancer peptide of the invention.

The invention also provides isolated cells transfected or transduced with the recombinant expression vector comprising the atom mentioned nucleic acid encoding a cancer peptide of the invention alone or in combination with a DNA sequence encoding at least one immunostimulatory molecule. The present invention also provides isolated cells transfected or transduced with the recombinant virus having incorporated into the viral genome or portion thereof the above-mentioned nucleic acid encoding a cancer peptide of the invention. The vectors and cells may serve as vaccines in which expression of a cancer peptide results in the stimulation of tumor antigen specific T lymphocytes in a mammal immunized with the vaccine.

The present invention provides a method of recombinantly producing a cancer peptide of the invention comprising:
a. inserting a nucleotide sequence of SEQ ID NO : 3 or portion or variant thereof, into an expression vector;
b. transferring the expression vector into a host cell;
c. culturing the host cell under conditions appropriate for expression of the cancer peptide or functional portion or derivative thereof; and
d. harvesting the recombinant cancer peptide, or functional portion or derviative thereof.

The present invention also provides an *in vitro* method of detecting the presence of cancer or precancer in a mammal comprising:
a. contacting a nucleic acid complementary to the portion of the nucleic acid sequence of SEQ ID NO: 3 encoding the peptide having the sequence of SEQ ID NO: 5, with a test biological sample of mRNA taken from the mammal under conditions allowing for a complex to form between the sequence and the mRNA;
b. detecting the complex;
c. comparing the amount of mRNA in the test sample with an amount of mRNA from a known normal biological sample, wherein an increased amount of mRNA from the test sample is indicative of cancer or precancer.

The present invention also provides an *in vitro* method of detecting the cancer peptide of the invention in a biological sample comprising:
a. contacting the sample with antibodies specific for said cancer peptide under conditions to form an immune complex, and
b. detecting the presence of the immune complex.

Still another aspect of the invention is a non-human transgenic animal carrying and expressing a gene consisting of SEQ ID NO:3 or a portion or derivative thereof, wherein said portion or derivative encodes a peptide immunologically recognized by antigen specific cytotoxic T lymphocytes specific for the peptide having the sequence of SEQ ID NO: 5 of Figure 3A. Such transgenic animals are useful for screening of therapeutic agents useful in treating cancer.

Another aspect of the present invention is an isolated cancer antigen specific human cytotoxic T lymphocyte elicited by the cancer peptide of the invention.

Still another aspect of the invention are isolated monoclonal and polyclonal antibodies or antigen binding portions thereof that bind to the cancer peptide encoded by SEQ ID NO:3, for use in diagnostic and detection assays. The monoclonal and polyclonal antibodies may be provided in the form of a kit alone, or along with other reagents commonly used in diagnostic and detection assays.

The present invention also provides a pharmaceutical composition comprising a recombinant virus having incorporated into the viral genome or portion thereof the above-mentioned nucleic acid encoding a cancer peptide of the invention, alone or in combination with an exogenous immunostimulatory molecule, chemotherapy drug, antibiotic, antifungal drug, antiviral drug or combination thereof, and a pharmaceutically acceptable carrier.

The present invention provides the use of an effective amount of a cancer peptide of the invention alone or in combination with an HLA molecule or a fragment thereof for the preparation of a medicament, wherein said amount is effective in preventing or inhibiting cancer in a mammal.

The present invention provides the use of a recombinant virus having incorporated into the viral genome or portion thereof the above-mentioned nucleic acid encoding a cancer peptide of the invention, alone or in combination with an immunostimulatory molecule, for the preparation of a medicament, for preventing or inhibiting cancer in a mammal.

The present invention provides the use of T lymphocytes for the preparation of a medicament, preferably for inhibiting melanoma in a mammal, wherein T lymphocytes are *in vitro* exposed to a cancer peptide of the invention alone or in combination with an MHC molecule, or a fragment thereof, for a time sufficient to elicit cancer peptide specific T lymphocytes.

Preferred embodiments of the subject-matter claimed in the present application are set forth in claims 2 to 4, 6, 9, 11 to 13, 19 to 22, 26, 28 to 30, 39, 40 and 43.

### Brief Description of the Figures

These and other objects, features, and many of the attendant advantages of the invention will be better understood upon a reading of the following detailed description when considered in connection with the accompanying drawings:

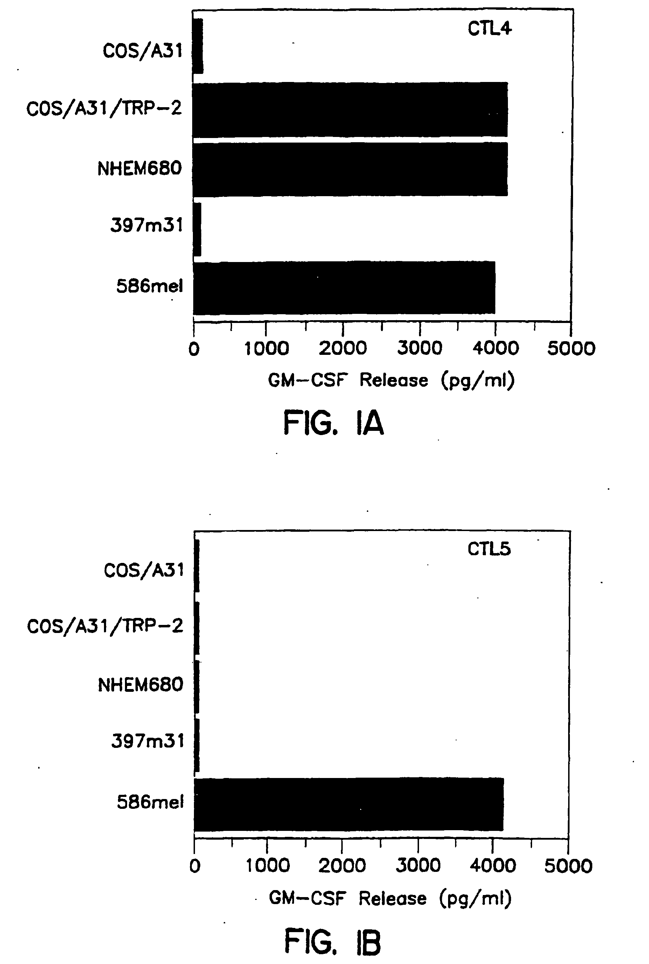

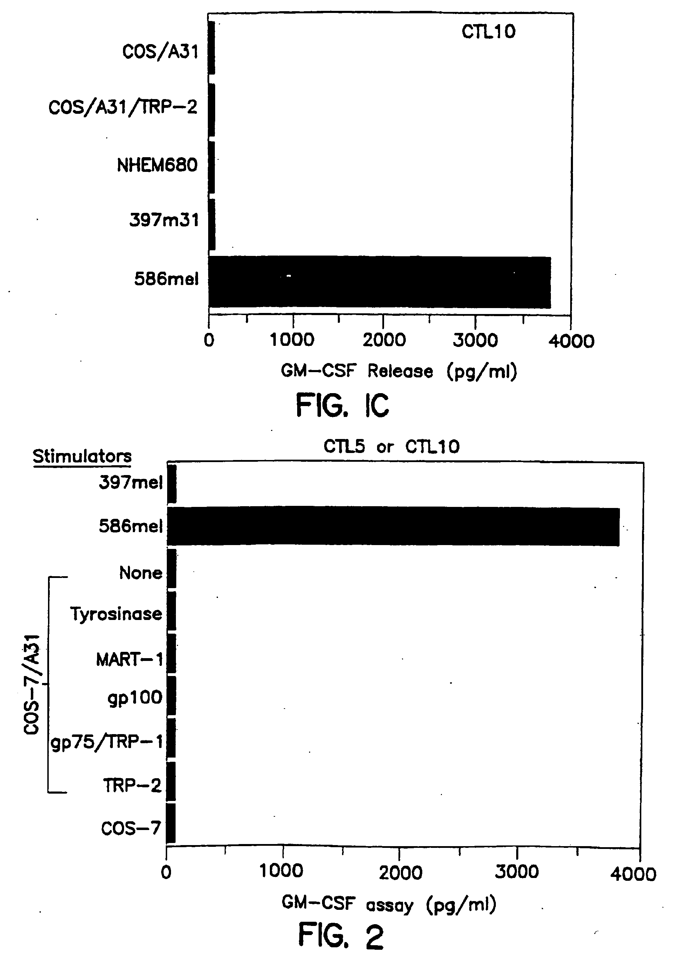

Figures 1A through 1C show the GM-CSF release of CTLs cloned from TIL586 in response to stimulation by various antigen expressing cells. CTL clones were isolated by the limiting dilution method and were further expanded. 1A: GM-CSF release by CTL clone 4 was measured after coculturing with different target cells. 1B and 1C: In a separate experiment, GM-CSF release by CTL clones 5 and 10 was determined after coculturing with different stimulators. NHEM680 is a HLA-A31-positive normal melanocyte cell line, 397mel is an HLA-A31-positive normal melanocyte cell line, 397mel is an HLA-A31-negative melanoma cell line and 586mel is an HLA-A31-positive melanoma cell line.
Figure 2 shows the GM-CSF release of CTL clone 5 and CTL clone 10 in response to stimulation by various known tumor antigens.
Figure 3A: Nucleotide and amino acid sequence of NY-ESO-1. Numbering of nucleotide sequence of *NY-ESO-1* starts from the first nucleotide in the 5' untranslated region. ORF1 represents the gene product of *NY-ESO-1,* and ORF2 represents a 58-amino acid gene product translated from the ORF2. Antigenic peptides recognized by CTL clone 2 and 5 are boxed. In addition, a peptide poorly recognized by CTL clone 5 is underlined.

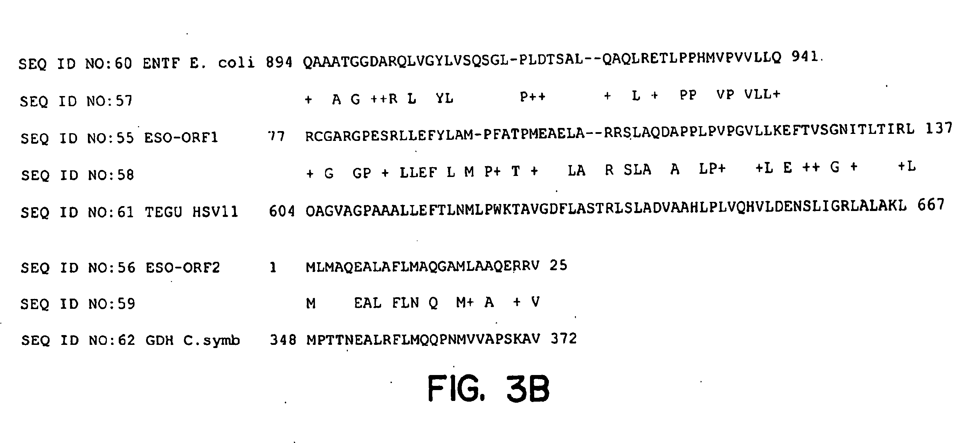

Figure 3B: Sequence alignment of the ESO-ORF1 protein with enterobactin synthetase component F (accession no. g250614) and tegument protein of herpes simplex virus type 1 (accession no. p10220). The residues are numbered with the first start site representing the first amino acid. Alignment of the ESO-ORF2 and glutamate dehydrogenase (accession no g1942184) is also shown. The conserved amino acid substitution is indicated by the + symbol.

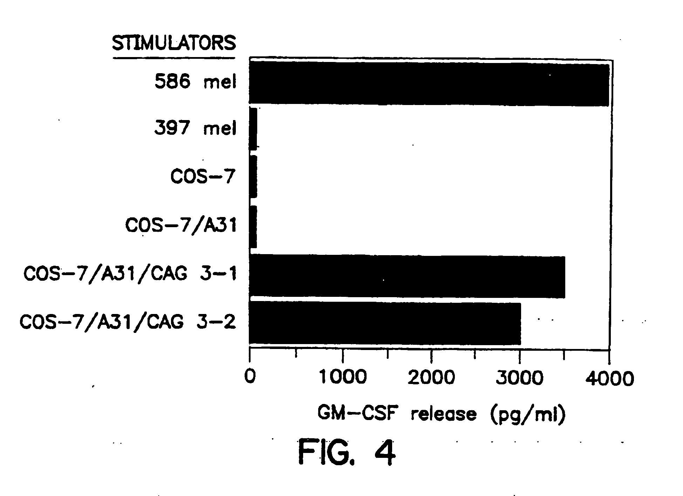

Figure 4 shows that COS-7 cells transfected with CAG-3 and an HLA-A31 molecule are recognized by CTL clone 5 lymphocytes.

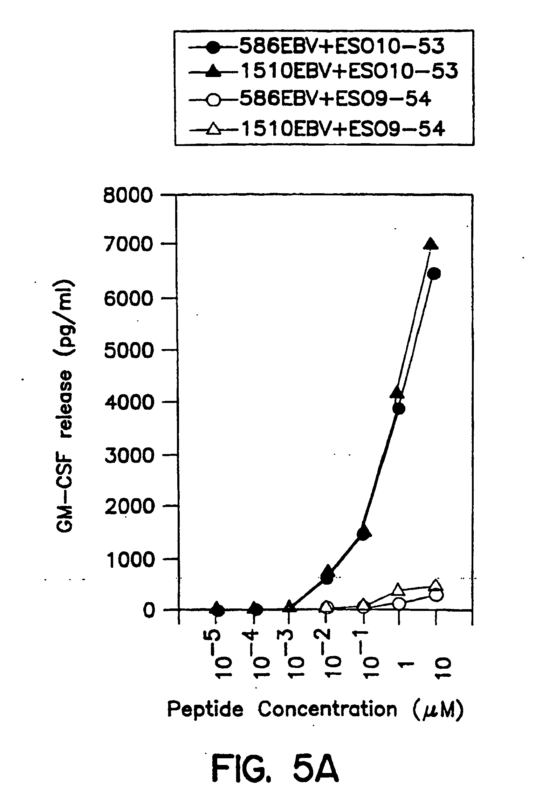

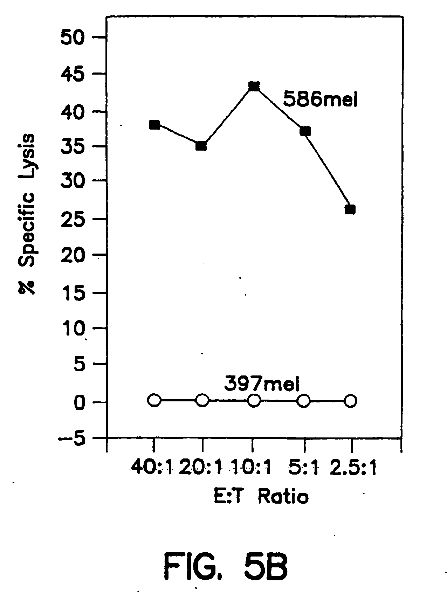

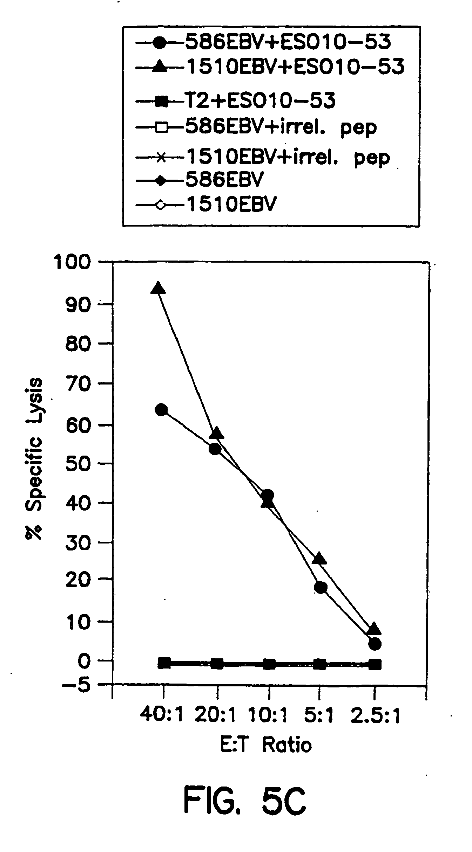

Figures 5A-5C: Characterization of the 9-mer ESO9-54 and 10-mer ESO10-53 derived from the normal open reading frame. Figure 5A: 586EBV or 15 10EBV B cells were pulsed with ESO10-53 and ES09-54 peptides at different concentrations for 120 min. After two washes with AIM-V medium with 120 IU IL-2. CTL clone 5 (1 x 10⁵/well) was added and incubated for 18 to 24 h. GM-CSF release by CTL clone 5 was determined by ELISA. Figure 5B: 586mel cells were lysed by CTL clone 5, but 397mel cells were not lysed by CTL clone 5 at different E:T ratios. Figure 5C: 586EBV and 1510EBV B cells were labeled with chromium overnight. The ESO10-53 peptide was then pulsed on the chromium-labeled 586EBV, 1510EBV, and T2 cells for 120 min. An irrelevant peptide containing an HLA-A31 peptide-binding motif was also pulsed on chromium-labeled 586EBV, 1510EBV B cells as negative controls. After peptide incubation and two washes, cytolysis of target cells by CTL clone 5 was determined in a 4-h chromium release assay. T2 pulsed with the ESO10-53 peptide was used for the specificity control.

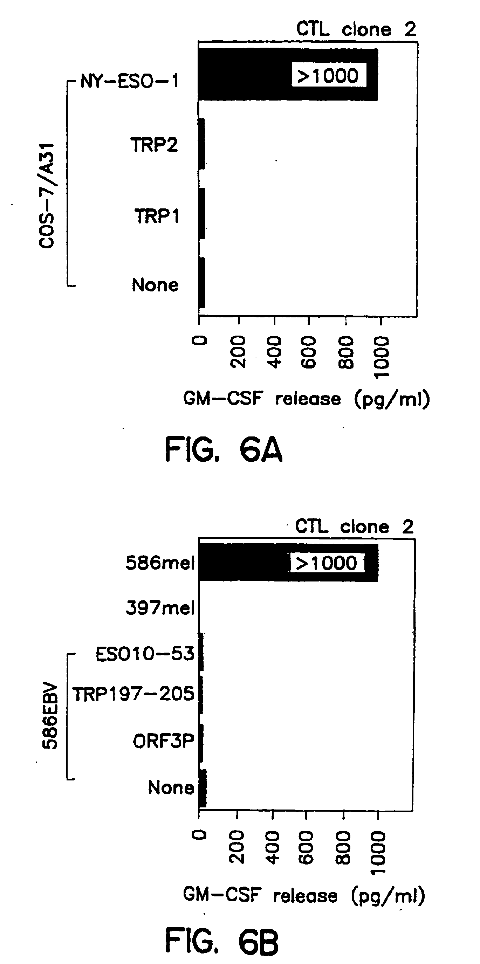

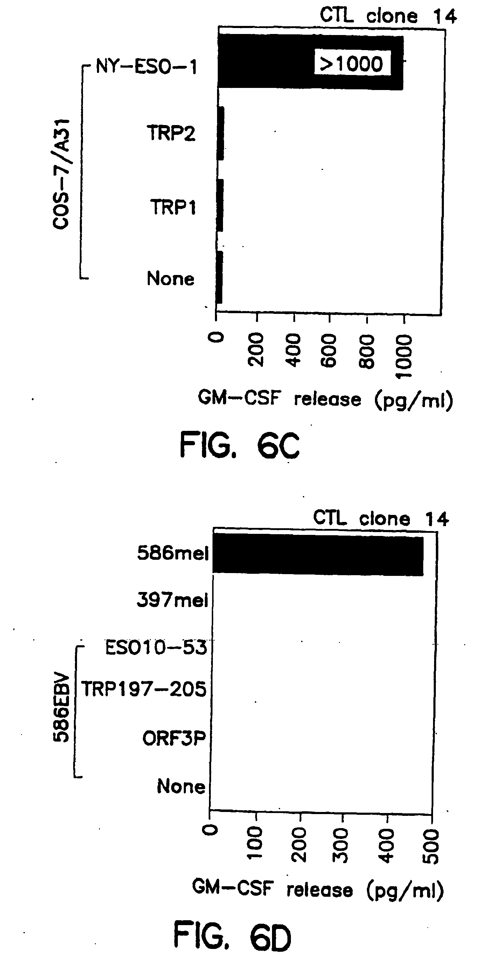

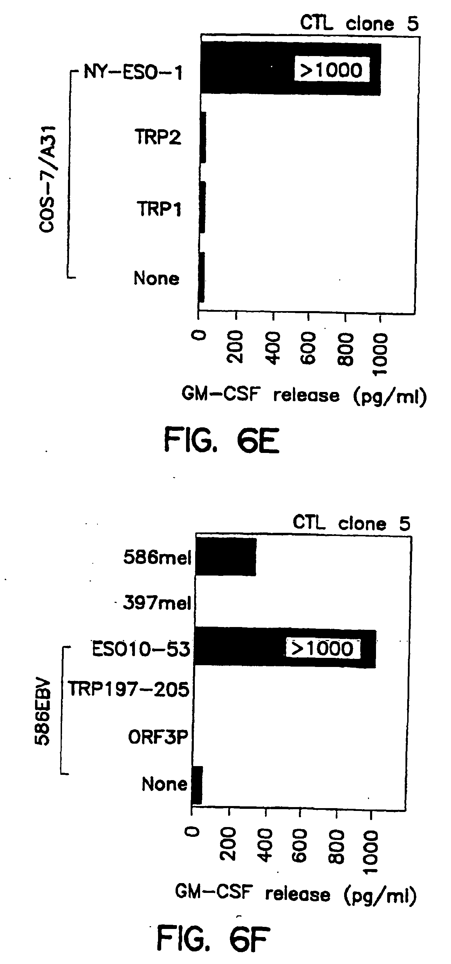

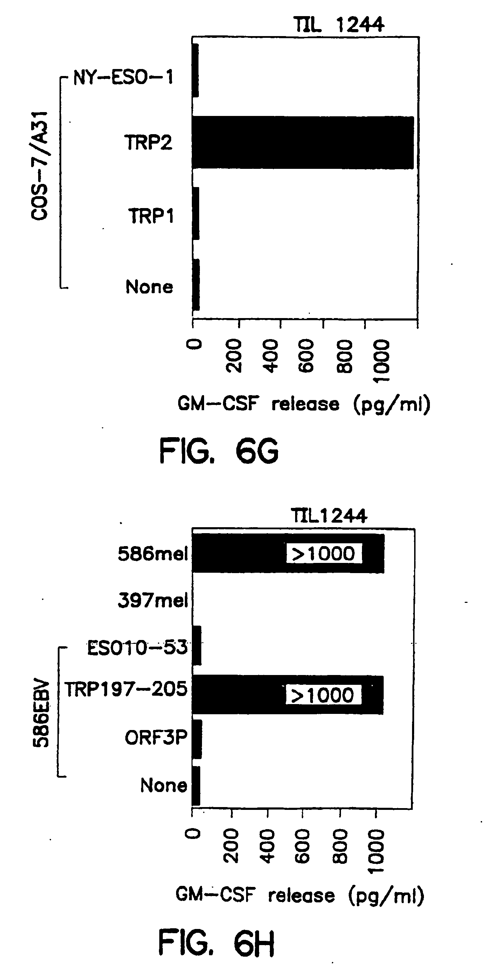

Figures 6A-6H. CTL clones 2 and 14 recognize the NY-ESO-1 gene, but not the ESO10-53 peptide derived from the NY-ESO-1 protein. CTL clones 2, 14 and 5 recognized NY-ESO-1 when cotransfected with HLA-A31 cDNA into COS-7 (Figure 6A, C, E and G). TIL1244, which recognized TRP-2 but not NY-ESO-1, was used as a specificity control. Figures 6B, D, F and H: CTL clones 2, 5, 14, and TIL1244 recognized 586mel. However, CTL clones 2 and 14 did not recognize the ESO10-53 peptide, whereas CTL clone 5 strongly recognized the ESO10-53 peptide when pulsed onto HLA-A31 positive 586EBV B cells. TIL1244 recognized the TRP197-205 peptide derived from TRP-2, 586EBV B cells alone or pulsed with the ORF3P peptide from an alternative reading frame of TRP1 were negative controls. 397mel is an HLA-A31-negative, NY-ESO-1-positive tumor line.

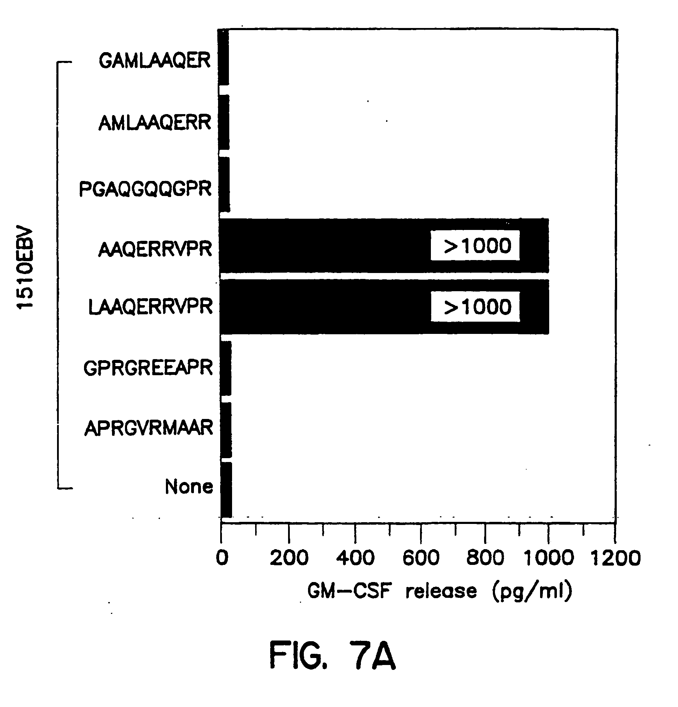

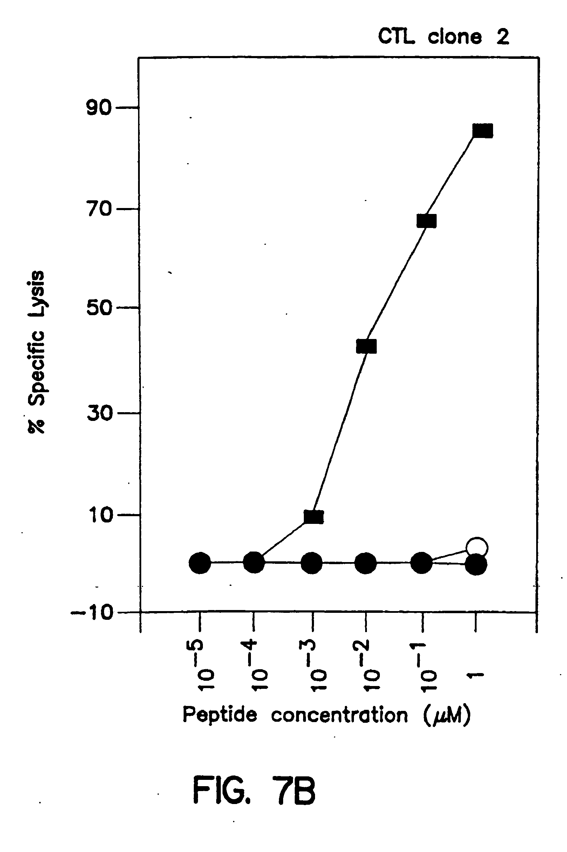

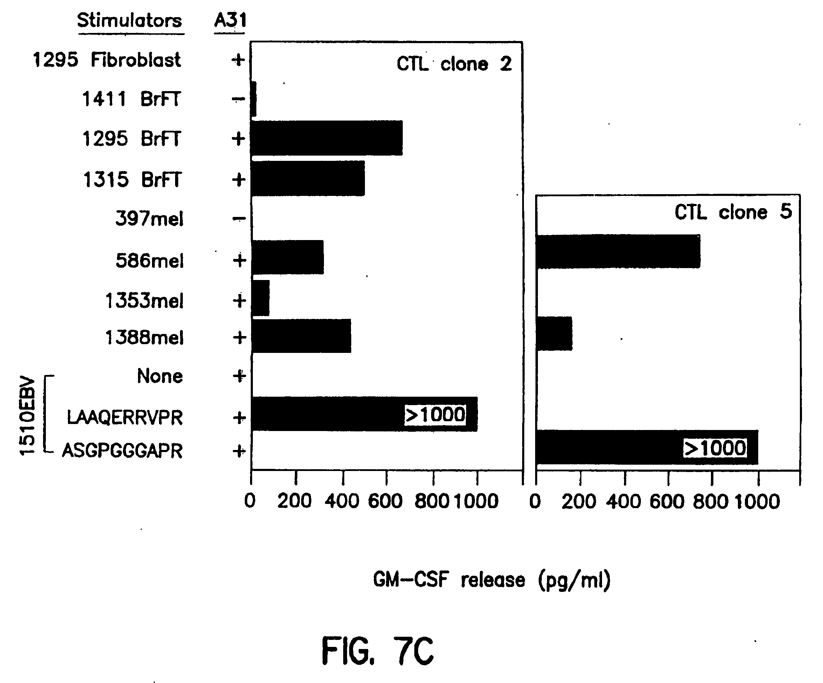

Figures 7A-7C: An alternative open reading frame of the *NY-ESO-1* gene and antigenic peptides recognized by CTL. Figure 7A: Identification of antigenic peptides from the ORF2. Thirty peptides were synthesized based on all potential ORFs and screened. Representative data are shown here. Seven peptides derived from ORF2 (see Fig. 3A) were pulsed on HLA-A31-positive 15 10EBV B cells and tested for T cell recognition based on GM-CSF release. 1510EBV alone was used as a negative control. Figure 7B: 1510EBV B cells were labeled with chromium for 2 h. The ESORF2-10-18 peptide was then pulsed on the chromium-labeled 1500EBV (solid square) and HLA-A31-negative 1102EBV (open circle) at different concentrations. 1510EBV pulsed with ESO10-53, which was recognized by CTL clone 5, was used for the specificity control (solid circle). After peptide incubation and three washes, cytolysis of target cells by CTL clone 2 was determined in a 4-h chromium release assay at an E:T ratio of 20:1. Figure 7C. Several tumor lines and fresh breast tumors were tested for recognition by CTL clone 2 to determine whether the ORF2 is translated in different tumors. 1510EBV B cells pulsed with ESO10-53, ORF2-10-18, or alone were included to evaluate the reactivity and specificity of CTL clone 2 and 5. Expression of HLA-A31 on the tumor cells is indicated.

### Detailed Description of the Invention

The present invention encompasses cancer peptides, portions and, derivatives thereof which are immunologically recognized by T lymphocytes of the immune system. The present invention further describes antigenic cancer epitope(s) which are contained in the cancer peptides. The antigenic cancer epitope specifically causes a cellular mediated immune response by interaction with T cells of the immune system. This interaction between the antigenic cancer epitope and the T cells causes the T cells to respond against, and prevent, eliminate or reduce the cancer in a mammal, including humans.

The cancer peptide and portions thereof are characteristically absent from or present in very low levels from normal cells from the majority of tissue sources and are present in high levels from pre-cancer and cancer cells. Expression of the cancer peptide at high levels correlates with transformation of normal cells to a pre-cancer or cancer cell.

The cancer peptides of the present invention form part of, or are derived from, cancers including but not limited to primary or metastatic melanoma, thymoma, lymphoma, sarcoma, lung cancer, liver cancer, non-Hodgkin's lymphoma, Hodgkin's lymphoma, leukemias, uterine cancer, cervical cancer, bladder cancer, kidney cancer and adenocarcinomas such as breast cancer, prostate cancer, ovarian cancer, pancreatic cancer, thyroid cancer and the like.

The term melanoma includes, but is not limited to, melanomas, metastatic melanomas, melanomas derived from either melanocytes or melanocyte related neyus cells, melanocarcinomas, melanoepitheliomas, melanosarcomas, melanoma in situ, superficial spreading melanoma, nodular melanoma, lentigo maligna melanoma, acral lentiginous melanoma, invasive melanoma or familial atypical mole and melanoma (FAM-M) syndrome.

Of particular interest are cancer peptides, fragments or derivatives thereof recognized by autologous CTL in patients with cancer, in particular melanoma. Of further interest are cancer peptides, fragments or derivatives thereof recognized by MHC (or HLA) restricted CTL, in particular MHC class I restricted CTLs:

The "tumor antigen" encompasses the cancer or tumor protein and any portion or peptide of the cancer or tumor protein capable of eliciting an anti-tumor response in mammals. In one embodiment, the tumor antigen includes the full-length gene product translated from the ORF 2 of the CAG-3 gene. In another embodiment, the tumor or cancer antigen is about 10 amino acids in length.

"Cancer peptides" as the term is used herein, encompasses any epitope or fragment of cancer or tumor protein, which acts as a tumor antigen.

The MHC restricted T lymphocytes are useful in identifying the gene product, CAG-3, associated with cancer and pre-cancer. In one embodiment, a cancer peptide, fragment or derivative thereof of the present invention comprises antigenic cancer epitope immunologically recognized by tumor infiltrating lymphocytes (TIL) derived from a cancer tumor of a mammal. Of particular interest are antigenic cancer epitopes recognized by cancer antigen specific cytotoxic T cells (CD 8⁺).

In one embodiment of the invention, the cancer peptide comprises the amino acid sequence: and a functional portion or derivative thereof.

In another embodiment, the cancer peptide comprises the amino acid sequence:
AAQERRVPR (SEQ. ID NO: 46).

In one embodiment, the cancer peptide comprises the amino acid sequence:
Position No.
12345678910
LAAQERRVPR (SEQ. ID NO: 47), and cancer epitopes,
fragments, derivatives and homologs thereof. Also included within the ambit of the present invention are cancer peptides of SEQ. ID NO: 47 having an amino acid substitution at position 1, 2, 6, 9 or combination thereof provided the substituted peptide retains functional activity in stimulation cancer peptide specific cytotoxic T lymphocytes.

Also included within the ambit of the present invention is a cancer peptide having one to about 10 additional amino acids, at the N-terminus of SEQ. ID NO: 47, preferably one to about 5 amino acids, more preferably one to about 3 amino acids at the N-terminus of SEQ. ID NO: 47. In one embodiment, cancer peptides of the present invention comprise the amino acid sequences:
MLAAQERRVPR (SEQ. ID NO: 48);
AMLAAQERRVPR (SEQ. ID NO: 49); and
GAMLAAQERRVPR (SEQ. ID NO: 50).

The CAG-3 cancer peptides and the antigenic cancer epitope contained within the tumor antigen of the present invention are expressed in normal testis but not in other normal tissue. The tumor antigen of the present invention present in significantly lower levels in most normal cells as compared to the elevated levels found in pre-cancer and cancer cells. Elevated expression of the tumor antigen correlates with transformation of normal cells to a pre-cancer or cancer cell. CAG-3 is expressed in a variety of cancers including melanoma, breast cancer, prostate cancer, ovarian cancer, lung cancer, thyroid cancer, bladder cancer and liver cancer.

Of further interest are CAG-3 cancer peptides, fragments or derivatives thereof recognized by MHC restricted CTL, in particular MHC class I restricted CTLs. HLA Class I restricted CTLs include but are not limited to HLA-A31, HLA-A3, HLA-A11, HLA-A33 and HLA-A68. A preferred HLA subtype recognized by the CAG-3 cancer peptides is the HLA-A31 subtype.

Another embodiment of the present invention encompasses derivatives and variants of the cancer peptides having sufficient homology to CAG-3 or epitopes thereof to effectively act as cancer peptides. Such peptides may have conservative amino acid changes at one or more positions. By conservative amino acid changes is meant, an amino acid change at a particular position which is of the same type as originally present; i.e. a hydrophobic amino acid exchanged for a hydrophobic amino acid, a basic amino acid for a basic amino acid, etc. Such amino acid changes do not significantly alter the overall charge and configuration of the peptide and therefore such variants maintain or enhance the anti-cancer activity of a cancer peptide. Examples of such conservative changes are well-known to the skilled artisan and are within the scope of the present invention.

The present invention also relates to functionally equivalent variants of the CAG-3 cancer peptides. "Functionally equivalent variants" includes peptides with partial sequence homology, peptides having one or more specific conservative and/or non-conservative amino acid changes, peptide conjugates, chimeric proteins, fusion proteins and peptide nucleic acids.

The CAG-3 cancer peptides, tumor antigen and their antigenic cancer epitopes may be purified and isolated from natural sources such as from primary clinical isolates, cell lines and the like. The CAG-3 cancer peptide and portions thereof are at least 90% pure, preferably at least 95% pure and as pure as 100%. The cancer peptides and their antigenic epitopes may also be obtained by chemical synthesis or by recombinant DNA techniques known in the arts. Techniques for chemical synthesis are described in J.M. Steward and J.D. Young, "Solid Phase Peptide Synthesis", W.H. Freeman & Co., San Francisco, 1969; M. Bodansky et al. "Peptide Synthesis", John Wiley & Sons, Second Edition, 1976, and J. Meienhofer, "Hormonal Proteins and Peptides", Vol. 2, p. 46, Academic Press, New York, 1983 and E. Schroder and K. Kubke, "The Peptides", Vol. 1, Academic Press, New York, 1965.

The CAG-3 cancer peptides and their antigenic cancer epitopes may be formulated with pharmaceutically acceptable carriers into pharmaceutical compositions by methods known in the art. The composition is useful as a vaccine to prevent or treat cancer. The composition may further comprise at least one immunostimulatory molecule. Immunostimulatory molecules to be used in conjunction with the cancer peptide or portion thereof for stimulating antigen specific T cell responses include but are not limited to one or more major histocompatibility complex (MHC) molecules, such as class I and class II molecules, preferably a class I molecule. The composition may further comprise other stimulator molecules including B7.1, B7.2, ICAM-1, ICAM-2, LFA-1, LFA-3, CD72 and the like, and cytokines which include but are not limited to IL-1 through IL-15, TNFα, IFNγ, RANTES, G-CSF, M-CSF, IFNα, CTAP III, ENA-78. GRO, I-309, PF-4, IP-10, LD-78, MGSA, MIP-1α, MIP-1β, or a combination thereof, and the like for immunopotentiation.

The stimulatory molecule may be provided as a physically separate entity or it may be provided in the membrane of an antigen presenting cell such as B-cell, macrophage or dendritic cell, in the membrane of a liposome, or expressed on the surface of a transduced or transfected cell. DNA sequences of MHC immunostimulatory molecules are available from GenBank and the like.

The CAG-3 cancer peptides, tumor antigen and their antigenic cancer epitopes are useful in preventing or treating cancer and useful in diagnostic assays for detecting cancer or precancer in a mammal, including humans. The cancer peptides or portions thereof may be in the form of a derivative in which other constituents are attached thereto such as radiolabels, biotin, fluorescein. A targeting agent may also be attached to the tumor antigen, cancer peptides or portions thereof that allow for specific targeting to a specific organ, tumor or cell types. Such targeting agents may be hormones, cytokines, cellular receptors and the like. The cancer peptide, tumor antigen and portions thereof may be prepared in the form of a kit, alone or in combination with other reagents.

Another aspect of the invention is a vaccine useful in inducing tumor-specific cell-mediated immunity against cancer.

Approaches to cancer immunotherapy can be divided into active or passive categories. Active immunotherapy involves the direct immunization of cancer patients with cancer antigens in an attempt to boost immune responses against the tumor. Passive immunotherapy refers to the administration of immune reagents, such as immune cells or antibodies with antitumor reactivity with the goal of directly mediating antitumor responses.

Most prior attempts at active immunotherapy utilized either intact cancer cells or cancer cell extracts with the expectation that these materials contained tumor antigens in an amount and form capable of stimulating immune responses. The molecular identification of cancer antigens however, has open new possibilities for developing immunotherapies for the treatment of human cancer. A summary of some of these approaches is presented in Table 1.

**Table 1 Cancer Therapies Based on the Molecular**

| Identification of Cancer Antigens | | | |
|---|---|---|---|
| 1. | Active immunotherapy with: | | |
| | a. | Immunodominant peptides | |
| | | 1) | alone |
| | | 2) | combined with adjuvants |
| | | 3) | linked to helper peptides, lipids or liposomes |
| | | 4) | pulsed onto antigen presenting cells |
| | b. | Immunodominant peptides with amino acids substitutions to increase binding to MHC molecules | |
| | c. | Proteins alone or combined with adjuvants | |
| | d. | "Naked" DNA encoding cancer antigens | |
| | | 1) | "gene gun" for intradermal injection |
| | | 2) | intramuscular injection |
| | | 3) | linked to lipids |
| | e. | Recombinant viruses such as vaccinia, fowlpox or adenovirus encoding | |
| | | 1) | cancer antigens alone |
| | | 2) | cancer antigens plus genes encoding cytokines, |
| | | | costimulatory molecules, or other genes to enhance the |
| | | | immune response |
| | f. | Recombinant bacteria such as BCG, Salmonella or Listeria encoding cancer antigens alone or in combination with immunostimulatory molecules | |
| 2. | Active immunotherapy (above) followed by the administration of immunostimulatory cytokines. | | |
| | 1. | IL-2 | |
| | 2. | IL-6 | |
| | 3. | IL-10 | |
| | 4. | IL-12 | |
| | 5. | IL-15, and the like. | |
| 3. | Passive immunotherapy with anti-tumor lymphocytes raised by in vitro sensitization of TIL or PBL to | | |
| | 1. | immunodominant peptides pulsed onto antigen presenting cells (raise CD8 cells) | |
| | 2. | antigenic proteins coincubated with antigen presenting cells (exogenous antigen presenting pathway to raise CD4 cells). | |

The insertion of the gene encoding CAG-3 cancer antigens into high efficiency expression systems such as E. coli, yeast or baculovirus and the like provides the opportunity to obtain large amounts of purified tumor antigen for use in immunization. Alternatively, the immunodominant peptides from these tumor antigens could readily be synthesized in vitro and purified in large amounts for immunization alone or in a form intended to improve their immunogenicity such as in combination with adjuvant, linkage to lipids/liposomes or helper peptides, or pulsed onto antigen presenting cells. Modification of individual amino acids of the immunodominant peptides to improve binding efficiency to MHC antigens can potentially increase immunogenicity compared to the native peptide.

Recent techniques utilizing "naked" DNA injected directly into muscle or into the skin have been shown to raise both cellular and humoral immune reactions to encoded antigens (Cooney, E.L., A.C. Collier, P.D. Greenberg, R.W. Coombs, J. Zarling, D.E. Arditti, M.C. Hoffman, S.L. Hu and L. Correy, 1991, *Lancet* 337:567; Wolff, J.A., R.W. Malone, P. Williams, W. Chong, G. Acsadi, A. Jani, and P.L. Felgner, 1990, *Science* 247:1465; Davis, H.L., RG. Whalen, and B.A. Demeniex, 1993, *Hum. Gene Ther.* 4:151; Yang, N.S., J. Burkholder, B. Roberts, B. Martinelli, and D. McCabe, 1990, *Proc. Natl. Acad. Sci. USA* 87:9568; Williams, R.S., S.A. Johnston, M. Riedy, M.J. DeVit, S.G. McElligott, and J.C. Sanford, 1991, *Proc. Natl. Acad. Sci. USA* 88:2726; Fynan, E.R., Webster, D.H. Fuller, J.R Haynes, J.C. Santoro, and H.L. Robinson, 1995, *Proc. Natl. Acad Sci. USA* 90:11478; Eisenbraum, M.D., D.H. Fuller, and J.R. Haynes, 1993, *DNA and Cell Bio.* 12:791; Fuller, D.H. and J.R. Haynes, 1994, *AIDS Res. Hum. Retrovir.* 10(11):1433; Acsadi, G., G. Dickson, D.R. Love, A. Jani, F.S. Walsh, A. Gurusinghe, J.A. Wolff, and K.E. Davies, 1991, *Nature* 352:815). Techniques using nonviable DNA vectors have the advantage of ease of preparation and safety of administration. The nucleic acid sequence of the present invention is useful as an immunogen and as a DNA vaccine against cancer. The nucleic acid sequence of the present invention of CAG-3 cancer peptides may be administered using a gene gun in amounts to elicit a cellular response against a cancer cell. Nomogram quantities are useful for such purposes.

An effective form of immunization involves the incorporation of genes encoding immunogenic molecules into recombinant bacteria such as BCG, Salmonella or Listeria or into recombinant viruses such as vaccinia, fowlpox or adenovirus and the like. The genes encoding CAG-3 cancer antigens can be expressed either alone or in combination with genes encoding immunostimulatory molecules or other genes which can enhance the immune response following infection. Studies with model tumor antigens in murine models have shown that incorporation of the gene for interleukin-2 (IL-2) or B7.1 can increase the immunogenicity of model tumor antigens and even mediate the regression of established lung metastases bearing these antigens. Active immunotherapy followed by the exogenous administration of immunostimulatory cytokines such as IL-2, IL-6, IL-10, IL-12, or IL-15 may also be used to improve immune responses.

Passive immunotherapy with genetically modified immune cells (commonly referred to as adoptive immunotherapy) capable of recognizing human tumor antigens is effective in mediating the regression of cancer in selected patients with metastatic melanoma. In vitro techniques have been developed in which human lymphocytes are sensitized in vitro to tumor antigen immunodominant peptides presented on antigen presenting cells. By repetitive in vitro stimulation cells can be derived with a far greater capacity to recognize human tumor antigens than the TIL that were used to clone the genes encoding these antigens. Thus by repeated in vitro sensitization with the cancer peptides, lymphocytes could be derived with 50 to 100 times more potency of TIL. The adoptive transfer of these cells may be more effective in mediating tumor regression in vivo than are conventionally grown TIL.

For preventing or inhibiting cancer, the cancer peptides or portions thereof may be administered via one of several routes including but not limited to intravenous, intramuscular, subcutaneous, intradermal, intraperitoneal, intrathecal, intrapleural, intrauterine, rectal, vaginal, topical, intratumor and the like.

Administration may be by transmucosal or transdermal means. For transmucosal or transdermal administration, penetrants appropriate to the barrier to be permeated are used in the formulation. Such penetrants are generally known in the art, and include, for example, for transmucosal administration bile salts and fusidic acid derivatives. In addition, detergents may be used to facilitate permeation. Transmucosal administration may be by nasal sprays, for example, or suppositories. For oral administration, the cancer peptide, tumor antigen, portion or variant thereof is formulated into a conventional oral administration form such as capsules, tablets and toxics.

In general, it is desirable to provide the recipient with a dosage of CAG-3 cancer peptide or portion thereof of at least about 1pg per Kg bodyweight, preferably at least about Ing per Kg bodyweight, more preferably at least about 1µg or greater per Kg bodyweight of the recipient. A range of from about 1ng per Kg bodyweight to about 100mg per Kg bodyweight is preferred although a lower or higher dose may be administered. The dose is effective to prime, stimulate and/or cause the clonal expansion of CAG-3 cancer antigen specific T lymphocytes, preferably cytotoxic T lymphocytes, which in turn are capable of preventing or inhibiting cancer in the recipient.

The dose is administered at least once and may be provided as a bolus or a continuous administration. Multiple administrations of the dose over a period of several weeks to months may be preferable. Subsequent doses may be administered as indicated.

For preventing cancer in a mammal, a vaccine comprising the CAG-3 cancer peptide or portion thereof is administered to the mammal in an amount effective to prevent cancer in the mammal. Of particular interest is a vaccine comprising SEQ. ID NO: 46,47 or combination thereof.

For reducing tumor burden in animals having tumors an effective amount of a CAG-3 antigenic cancer epitope is administered at a site of tumor burden, said amount is effective to reduce the size of the tumor at the site.

Autologous cytotoxic lymphocytes or tumor infiltrating lymphocytes may be obtained from a patient with cancer. The lymphocytes are grown in culture and cancer antigen specific lymphocytes expanded by culturing in the presence of specific cancer peptides or antigenic cancer epitopes alone or in combination with at least one immunostimulatory molecule with cytokines. The antigen specific lymphocytes are then infused back into the patient in an amount effective to reduce or eliminate the tumors in the patient.

After immunization the efficacy of the vaccine can be assessed by production of immune cells that recognize the CAG-3 cancer antigen, as assessed by specific lytic activity, specific cytokine production, tumor regression or combination of these. If the mammal to be immunized is already afflicted with cancer or metastasis cancer the vaccine can be administered in conjunction with other therapeutic treatments such as immunomodulators, for example, IL-2, IL-6, IL-10, IL-12, IL-15, interferon, tumor necrosis factor and the like, chemotherapeutic drugs such as cisplatinum, antiviral drugs such as gancyclovir, amphotericin B, antibiotics and the like.

Another aspect of the invention is a DNA sequence encoding a cancer peptide of the invention, wherein the DNA sequence does not encode SEQ ID NO: 4 of Figure 3A.

In one embodiment, the DNA sequence comprises SEQ. ID NO.: 3, a portion thereof and functionally equivalent sequence variant thereof that encode a CAG-3 cancer peptide or portions thereof recognized by CAG-3 cancer antigen specific T lymphocytes including tumor infiltrating lymphocytes. Also encompassed by the present invention are nucleic acid sequences complementary, as well as anticomplementary to SEQ. ID NO: 3.

In another embodiment, the DNA sequence comprises:
CCT CGG GGC CGG GAG GAG GCG CCC CGC GGG (SEQ. ID NO: 51).

In another embodiment, the DNA sequence of the present invention encodes the cancer peptide LAAQERRVPR of SEQ. ID NO: 47, the cancer peptide, AAQERRVPR of SEQ. ID NO: 46, cancer epitopes, fragments, derivatives or homologs thereof.

In one embodiment, the DNA sequence comprises:
CTG GCG GCC CAG GAG AGG CGG GTG CCA CGG (SEQ. ID NO: 52) and variants thereof.

In another embodiment, the DNA sequence comprises:
GCG GCC CAG GAG AGG CGG GTG CCA CGG (SEQ. ID NO: 53)

Due to degeneracy in the generic code, variations in the DNA sequence will result in translation of an equivalent cancer peptide. As a result, substitutions are included in the ambit of the invention as long as the substitution results in expression of a cancer peptide that is recognized by antigen specific HLA-restricted T cells specific for the peptide having sequence of SEQ ID NO:5.

All or part of the open reading frame DNA sequence from the CAG-3 gene encoding the peptide having the sequence of SEQ ID NO:5 may be used as probes to identify and isolate the homologs of the cancer peptide in other mammalian species. In one embodiment, a human cDNA sequence is used to screen a mammalian cDNA library for a murine homolog nucleic acid sequence. Positive clones are selected and sequenced. Examples of tissue sources from which the cDNA library can be synthesized include but are not limited to dermis, epidermis, solid tumors, melanomas, melanocytes, and the like. One skilled in the art will understand the appropriate hybridization conditions to be used to detect the homologs. Conventional methods for nucleic acid hybridization construction of libraries and cloning techniques are described in Sambrook et al, (eds) (1989) in "Molecular Cloning. A Laboratory Manual" Cold Spring Harbor Press, Plainview, New York and Ausubel et al.(eds) in "Current Protocols in Molecular Biology" (1987), John Wiley and Sons, New York, New York.

Another aspect of the invention are nucleic acid probes for the detection and quantification of RNA that transcribes the cancer peptides of the invention in biological samples isolated from a mammal with cancer. Alterations in the level of RNA relative to a control RNA sample is useful in diagnosis and prognosis of the disease in the mammal.

In one embodiment, mRNA is derived from tissue of a patient suspected of having cancer or precancer and compared with mRNA derived from a healthy control subject. A quantitative and/or qualitative increase of the mRNA encoding a cancer peptide of the invention in the patient, as compared to the control, is indicative of cancer or precancer in the patient. The mRNA may be detected using oligonucleotide probes hybridizable with the mRNA. In one embodiment the probe is hybridizable with the transcription product of SEQ. ID NO: 51.

Combinations of oligonucleotides pairs based on the sequence encoding the cancer peptide of the invention may be used as PCR primers to detect mRNA in biological samples using the reverse transcriptase polymerase chain reaction (RT-PCR) process for amplifying selected RNA sequences. The present invention also encompasses in situ PCR and in situ RT-PCR for detection of DNA and RNA encoding the cancer peptides of the invention. The technique is preferred when the copy number of a target nucleic acid is very low, or when different forms of nucleic acids must be distinguished. The method is especially useful in detecting and differentiating precancer and cancer cells from normal cells.

The present invention also encompasses a recombinant expression vector comprising the DNA sequence encoding a cancer peptide of the invention. Optionally the vector may also comprise a DNA sequence encoding at least one immunostimulatory molecule. The vector may also contain a gene encoding green fluorescent protein for use in detecting localization of CAG-3 cancer peptides in cells and tissues.

Eukaryotic expression vectors include but are not limited to retroviral vectors, vaccinia virus vectors, adenovirus vectors, herpes virus vectors, fowlpox virus vectors, baculovirus vectors, human papillomavirus vectors, equine encephalitis vectors, influenza virus vectors and the like.

The present invention encompasses a recombinant virus expressing a CAG-3 cancer peptide of the invention. The recombinant virus may also express at least one immunostimulatory molecule. The recombinant virus is capable of eliciting or upregulating a cell-mediate immune response in a mammal for the purpose of preventing or treating cancer in the mammal, particularly humans.

A host cell infected with the recombinant virus expresses the cancer peptide of the invention, alone or in combination with at least one immunostimulatory molecule. The host cell may also be infected with a recombinant virus expressing an HLA class I molecule.

Methods for constructing and expressing exogenous gene products from recombinant vaccinia virus vectors are disclosed by Perkus et al. Science 229:981-984, 1985, Kaufman et al.Int. J. Cancer 48:900-907, 1991, Moss Science 252:1662, 1991, Smith and Moss BioTechniques Nov/Dec, p. 306-312,1984, and U.S. Patent No. 4,738,846. Sutter and Moss (Proc. Natl. Acad. Sci. U.S.A. 89:10847-10851, 1992) and Sutter et al (Virology 1994) disclose the construction and use as a vector, the non-replicating recombinant Ankara virus (MVA, modified vaccinia Ankara) which may be used as a viral vector in the present invention. Baxby and Paoletti (Vaccine 10:8-9, 1992) disclose the construction and use as a vector, a non-replicating poxvirus, including canarypox virus, fowlpox virus and other avian species for use as a viral vector in the present invention.

The vectors of the present invention may be placed in an appropriate host cell for the expression of the cancer peptide of the invention or antigenic cancer epitope thereof. Eukaryotic host cell lines include, but are not limited to COS cells, CHO cells, Hela cells, NIH/3T3 cells, insect cells, antigen presenting cells such as dendritic cells and the like. Optionally the host cell may also express a stimulatory molecule. In the case where the host cells express both the cancer peptide or antigenic cancer epitope in combination with at least one MHC (or HLA) molecule, it is preferable that a eukaryotic expression system be used to allow for proper glycosylation. The expression of both the cancer antigen and the immunostimulatory molecule by the host cell provides the necessary MHC restricted peptide to specific T cells and the appropriate signal to the T cell to aid in antigen recognition and proliferation or clonal expansion of antigen specific T cells. The overall result is an upregulation of the immune system. The upregulation of the immune response is manifest by an increase in cancer antigen specific cytotoxic lymphocytes which are able to kill or inhibit the growth of cancer or precancer cells.

The DNA may be inserted into the host cell by transfection, transduction, liposomes and the like by methods known in the art. (Sambrook et al., 1989, in: "Molecular Cloning A Laboratory Manual", Cold Spring Harbor press, Plainview, New York). For liposomes, cationic lipids are preferred, for example, polycationic lipid, dimyristyloxypropyl-3-dimethyl-hydroxyethyl ammonium (DMRIE) complexed with the neutral phospholipid dioleoyl phosphatidylethanolamine (DOPE) as disclosed by Nabel, E.G. et al., 1992, Hum. Gene. Ther. 3:367-275; Nabel, GJ. et al., 1992, Hum. Gene Ther. 3:649-656; Stewart, MJ. et al. 1992 Hum. Gene Ther. 3:399-410; Nabel, GJ. et al. 1993 Proc. Natl. Acad. Sci. USA 90:11307-11311; and Harrison, G.S. et al. 1995 Bio Techniques 19:816-823.

The recombinant cancer protein of the invention, tumor antigen or antigenic cancer epitope expressed by the host cells may be purified from cell lysates or cell supernatants by standard protein purification procedures known in the art. These include but are not limited to molecular sieve chromatography, ion-exchange chromatography, isoelectric focusing, gel electrophoresis, affinity chromatography, HPLC, reverse phase HPLC and the like. (Ausubel et al., 1987, in Current Protocols in Molecular Biology, John Wiley and Sons, New York, NY). Immunoaffinity chromatography may also be used for purification using anti-cancer protein antibodies or antigen binding fragments thereof as described herein, as the immunoaffinity agent.

The recombinant virus may also be used as a therapeutic or vaccine. In such uses it is desirable to provide the recipient with a dosage of recombinant virus in the range of from about 10⁵ to about 10¹⁰ plaque forming units/mg mammal, although a lower or higher dose may be administered.

The recombinant viral vector may be introduced into a mammal either prior to any evidence of cancer such as melanoma or to mediate regression of the disease in a mammal afflicted with a cancer such as melanoma. Examples of methods for administering the viral vector into mammals include, but are not limited to, exposure of cells to the recombinant virus ex vivo, or injection of the recombinant virus into the affected tissue or intravenous, subcutaneous, intradermal, intramuscular and the like administration of the virus. Alternatively, the recombinant viral vector or combination of recombinant viral vectors may be administered locally by direct injection into the cancerous lesion or topical application in a suitable pharmaceutically acceptable carrier. The quantity of recombinant viral vector, carrying the nucleic acid sequence of interest is based on the titer of virus particles. A preferred range for immunization is about 10⁵ to 10¹⁰ virus particles per mammal, preferably a human.

The invention provides a non-human transgenic animal which has incorporated into its genome one or more copies of the DNA sequence encoding a cancer peptide of the invention. The general method of producing transgenic animals is described in Krimpenfort et al.U.S. Patent No. 5,175,384, Leder et al.U.S. Patent No. 5,175,383, Wagner et al.U.S. Patent No. 5,175,385, Evans et al.U.S. Patent No. 4,870,009 and Berns U.S. Patent No. 5,174,986. The incorporation of the gene results in overexpression, altered expression or expression of multiple forms or variants of the cancer peptides. The resulting transgenic animal is useful in studies of the development of cancer or tumor antigen of the present invention. The animal model is useful in screening vaccines and chemotherapeutic drugs for cancer treatment. The transgenic animal is also useful in studies of the development of cancer.

This invention further comprises an isolated antibody or antigen binding portion thereof elicited by immunization of the cancer peptide of the present invention. In the case where the cancer peptide is comprised of only a few amino acids, the cancer peptide may be conjugated to a carrier protein in order to elicit an antibody response. Carrier proteins such as KLH, tetanus toxoid and the like and methods of conjugation are known in the art. The antibody has specificity for and reacts or binds with the cancer peptide of the present invention.

Exemplary antibody molecules are intact immunoglobulin molecules, substantially intact immunoglobulin molecules or these portions of an immunoglobulin molecule that contain the antigen binding site, including those portions of immunoglobulin molecules known in the art as F (ab), F (ab'), F (ab')₂, humanized chimeric antibody, and F (v). Polyclonal or monoclonal antibodies may be produced by methods known in the art. (Kohler and Milstein (1975) Nature 256, 495-497; Campbell "Monoclonal Antibody Technology, the Production and Characterization of Rodent and Human Hybridomas" in Burdon et aL(eds.) (1985) "Laboratory Techniques in Biochemistry and Molecular Biology", Vol. 13, Elsevier Science Publishers, Amsterdam). The antibodies or antigen binding fragments may also be produced by genetic engineering. The technology for expression of both heavy and light chain genes is the subject of the PCT patent applications: publication number WO 901443, WO 9014424, Huse et al.(1989) Science 246:1275-1281, and U.S. Patent No. 4,946,778. Humanized immunoglobulins having one or more complementary determining regions and methods of making the antibodies are disclosed in U.S. Patent No. 5,585,089 and 5,530,101.

In one embodiment, the antibodies of the invention are used in immunoassays to detect cancer peptides of the invention in biological samples. The antibodies or antigen binding fragments thereof may be used to detect cancer peptides in tissue biopsy samples from a mammal afflicted with cancer. Assessment of the cancer antigen in a diseased tissue can be used to prognose the progression of the disease in a mammal or may diagnose the efficacy of a treatment. The immunoassay may be a radioimmunoassay, Western blot assay, immunofluorescent assay, enzyme immunoassay, chemiluminescent assay, immunohistochemical assay and the like and may be performed in vitro, in vivo or in situ. Standard techniques known in the art for ELISA are described in "Methods in Immunodiagnosis", 2nd Edition, Rose and Bigazzi, eds. John Wiley & Sons, 1980; Campbell et al. "Methods and Immunology", W.A. Benjamin, Inc., 1964; and Oellerich, M. 1984, J. Clin. Chem. Clin. Biochem. 22:895-904. Conventional methods for immunohistochemistry are described in Harlow and Lane (eds) (1988) In "Antibodies A Laboratory Manual", Cold Spring Harbor Press, Cold Spring Harbor, New York; Ausbel et al. (eds) (1987) In Current Protocols In Molecular Biology, John Wiley and Sons (New York, NY). Biological samples appropriate for such detection assays include but are not limited to cells, tissue biopsy, whole blood, plasma, serum, sputum, cerebrospinal fluid, pleural fluid, urine and the like.

The antibodies or antigen binding fragments of the present invention may also be used in immunotherapy. The antibodies or antigen binding fragment thereof is provided to a mammal in an amount sufficient to prevent, lessen or attenuate the severity, extent or duration of the cancer.

While the invention is described above in relation to certain specific embodiments, it will be understood that many variations are possible, and that alternative materials and reagents can be used without departing from the invention. In some cases such variations and substitutions may require some experimentation, but will only involve routine testing.

These examples which are not encompassed by the claims are for comparative purposes only.

### Example 1

### Materials and Methods

### Chemicals and Reagents

The following chemicals and reagents were purchased from the sources indicated: RPMI 1640, AIM-V media, Lipofectamine, G418 (GIBCO BRL, Gaithersberg, MD); the eukaryotic expression vector pCR3 (Invitrogen, San Diego, CA); anti-HLA-A31 monoclonal antibody (One lambda, Canoga Park, CA); anti-immunoglobulin M antibody conjugated with fluorescein isothiocyanate (Vector Laboratories, Inc., Burlingame, CA).

### Cytotoxic T lymphocytes (CTLs) and cell lines

TIL 586 were isolated from the tumor specimen of a patient with metastatic melanoma and grown in medium containing IL-2 (6000 IU/ml) (Chiron) for 32-60 days as previously described (Topalian, S., D. Solomon, F. P. Avis, A. E. Chang, D. L. Freeksen, W. M. Linehan, M. T. Lotze, C. N. Robertson, C. A. Seipp, P. Simon, C. G. Simpson, and S. A. Rosenberg, 1988, *J. Clin. Oncol.* 6:839-53). TIL586 and TIL1244 were predominantly CD8⁺ T cells. TIL1200 were grown under the same conditions as described for TIL586. TIL1244 recognized the TRP-2 peptide in the context of HLA-A31 and -A33 (31). The T cell clones or cloids were generated by limiting dilution methods (at 1 cell/well) from the TIL586 cell line, using allogeneic PBL (1 x 10³ cells/well) as feeder cells in RPMI1640 containing 10% human AB sera and 500 IU IL-2. After 12 days, the T cell clones were expanded in AIM-V medium containing 6000 IU/ml IL-2. To obtain an optimal expansion, we used the OKT3 expansion method, described by S. Riddell (32). Briefly, on day 0, 5 x 10⁴ to 5 x 10⁵ T cells were cocultured with HLA-A31⁺ PBL (500:1, PBL:T cell ratio) and 586EBV B cells (100:1, EBV:T cell ratio) in 25 ml of RPMI 1640 containing 11% human sera, 30 ng/ml OKT3 Ab, and antibiotics. On day 1, IL-2 was added at a final concentration of 180 IU/ml. The medium was changed with fresh medium containing 11% human sera and 180 IU/ml IL-2 on day 5. The medium was then changed every 3 days. On days 12 through 14, T cells were harvested, counted and cryopreserved.

Melanoma cell lines 397mel, 397mel/A31, 586mel, 624mel, 624mel/A31 and EBV transformed B-cell lines 586EBV and 1510EBV were established in this laboratory and cultured in RPMI 1640 medium containing 10% fetal calf serum (FCS). Normal cultured melanocytes derived from infant foreskin (NHEM680 purchased from Clonetics, CA) were cultured in melanocyte growth medium (MGM; Clonetics, CA). The COS-7 cell line was provided by Dr. W. Leonard (NIH).

295Br and 1315Br, fresh cryopreserved breast tumor digests, were cleaned with Ficoll gradient before use in T cell assays; 1295 fibroblast cells from the autologous patient were cultered for 22 to 64 days. 1315Br, culture A, were breast tumor cells grown in immunodeficient mice and then cultured in keratinocyto-SFM/2% FCS medium (Life Technologies), and 1315 Br, culture B, were grown in Hams F12/5% FCS for 77 to 80 passages; these cells were kindly provided by Dr. Stephen Ethier, University of Michigan, Ann Arbor, MI. 1398Br was an human papillomavirus (HPV) E6/E7-immortalized breast tumor line established in the Surgery Branch, National Cancer Institute. Prostate tumor lines 1535Pro, 1542Pro, and 1510 fibroblast were HPV E6/E7-immortalized cell lines.

### cDNA library construction

Total RNA was extracted from 586mel using Trizol reagent (Life Technologies. Poly(A) RNA was purified from total RNA by the poly AT tract system (Promega, Madison, WI) and then converted to cDNA using a cDNA construction kit (Life Technologies) with an oligo(dT) primer containing a *NotI* site. The cDNA was ligated to *BstXI* adaptors and digested with *Not*I, then ligated to the expression vector pcDNA3.1. The cDNA library was electroporated into DH10B cells (Life Technologies). Plasmid DNA pools, each consisting of ~ 100 cDNA clones, were prepared from bacteria.

### cDNA Library Screening and GM-CSF Secretion Assay

DNA transfection and GM-CSF assay were done as previously described (Wang, R. F., P. F. Robbins, Y. Kawakami, X. Q. Kang, and S. A. Rosenberg, 1995, *J. Exp. Med.* 181:799-804). Briefly, 200 µg of DNA carrying a different fragment and 50 ng of the HLA-A31 DNA were mixed with 2 µl of lipofectamine in 100 µl of senun-free DMEM for 15-45 min. The DNA/lipofectamine mixture was then added to the COS-7 (5 X 10⁴) cells and incubated overnight. The following day, cells were washed twice with DMEM medium. TIL586, CTL clone 5 or CTL clone 10 were added at a concentration of 5 X 10⁴ cells/well in AIM-V medium containing 120 IU/ml of IL-2. After 18-24 h incubation, 100 µl of supernatant was collected and GM-CSF was measured in a standard ELISA assay (R + D Systems, Minneapolis, MN). For peptides, 586EBV, 1510EBV and T2 cells were incubated with peptides at 37°C for 90 min, and then washed three times with AIM-V medium containing 120 IU/ml of IL-2. T cells were added and incubated for additional 18-24 h, 100 µl of supernatant was collected for GM-CSF assay. In some experiments, IFN-γ release assays were done using a standard ELISA kit (Endogen, Woburn, MA).

### Northern Blot Analysis

Total RNA was isolated by the guanidine isothiocyanate/cesium chloride centrifugation method. Total RNA from normal human tissue was purchased from Clontech (Palo Alto, CA). Twenty micrograms of total RNA was subjected to electrophoresis in a 1.2% formaldehyde agarose gel and transferred to a nylon membrane. An 0.8-kb DNA fragment of the *NY-ESO-1* gene was labeled with [α-³²P]CTP by the random priming method. Prehybridization and hybridization were performed according to the QuickHyb protocol (Stratagene, La Jolla, CA). Membranes were washed twice with 2 x SSC/0.1 % SCD at room temperature for 15 and twice with 0.1 x SSC/0.1% SDS at 60° for 30 min. The autoradiography was performed at -70°C.

### Reverse Transcriptase-PCR

Total RNA was extracted from tumor cell lines as described above. Five hundred nanograms of total RNA was used for conversion of RNA to cDNA by avian myeloblastosis virus (AMV) reverse transcriptase. cDNA was then amplified by PCR using the ESO-P2 (5'GCGGCTTCAGGGCTGAATGGATG) and ESO-P5 (5'-AAGCCGTCCTCCTCCAGCGACA) primers and One-Step RT-PCT system (Life Technologies). PCR products were amplified under denaturation conditions at 94°C for 30 s, annealing at 55° for 30 s, extension at 72° for 3 min for 40 cycles, and final elongation at 72° for 10 min. PCR products were analyzed on a 3% agarose gel.

### Cytotoxic lysis assays

Cytolytic assay was done as previously described (Kawakami, Y et al., 1994, *Proc. Natl. Acad. Sci.* USA 91:6458-62). Briefly, the target cells were labeled with chromium for 90 min. After washing three times, the cells were incubated with peptides at a concentration of 1 µg/ml for 90 min. The cells were washed again, counted, and then mixed with TIL586 or CTL clone 5 at the indicated ratio of effector : targets (E : T). Chromium release was measured after 4 h incubation. The peptides were synthesized by a solid-phase method using a peptide synthesizer (Model AMS 422, Gilson Co., Inc., Worthington, OH). Some peptides were purified by HPLC and had greater than 98% in purity. The mass of some peptides was confirmed by mass spectrometry analysis. For titration of the CAG-3 peptide recognized by TIL586, 586EBV B cells were incubated with various concentrations of the purified CAG-3 peptide or portion thereof. Percentage of specific lysis was determined from the equation (A-B)/(C-B) X 100 where A is lysis of 586EBV B cells by TIL586 or clone 5 in the presence of a peptide, B is spontaneous release from 586EBV B cells in presence of the same peptide but in the absence of effector cells, and C is the maximum chromium release. Cold target inhibition of cytolysis was performed using ⁵¹Cr-labeled 586mel or 624mel cells as "hot" targets and 586EBV B and T2 cells pulsed with peptides as "cold" targets.

### Example 2

### In vivo Protection Assay

For in vivo protection studies, MHLA-A31⁺ transgenic mice are immunized with 0, 1pg, 1ng, 1µg, 1mg or 100mg of cancer peptide (SEQ. ID NO: 4, 5, 14, 25, 34-38, 41, 42, 46 or 47), intravenously at day zero and day 14 before a subcutaneous challenge with 10⁴ CAG-3 B 16 mouse melanoma cells or intravenous challenge with 5 x 10⁵ CAG-3 B16 mouse melanoma cells. Mice receiving tumor cells subcutaneously are observed twice a week for tumor development and the size determined. Mice receiving tumor cells intravenously are euthanized on day 12 and the number of lung metastases determined as described by Houghton, A.N. 1994 J. Exp. Med. 180:1-40.

### Example 3

### In vivo Treatment Assay

For in vivo treatment, MHL-A31⁺ transgenic mice are challenged with either 1 x 10⁵ or 5 x 10⁵ CAG-3 B16 mouse melanoma cells intravenously in order to establish pulmonary metastases. Mice are subsequently vaccinated with a recombinant virus expressing cancer peptide (SEQ. ID NO: 4, 5, 14, 25, 34-38, 41, 42, 46 or 47) at 10⁵ PFU/mg body weight. Mice are euthanized on day 12 and the number of pulmonary metastases in vaccinated mice vs. non-vaccinated mice determined.

### Example 4

### Cancer Antigen Specific T Lymphocytes

### Immunotherapy

T-lymphocytes presensitized to a melanoma antigen may be effective in therapeutically treating mammals afflicted with a melanoma. T-lymphocytes are isolated from peripheral blood or melanoma tumor suspensions and cultured in vitro (Kawakami, Y. et al., 1988, J. Exp. Med. 168:2183-2191).

The T lymphocytes are exposed to the cancer peptide (SEQ. ID NO: 4, 5, 14, 25, 34-38, 41, 42, 46 or 47) at a concentration of 1 µg/ml alone or in the presence of IL-2, resensitized and expanded in culture. T-lymphocytes exposed to the cancer peptide are administered to a mammal at about 10⁹ to 10¹² lymphocytes per mammal. The lymphocytes are administered either intravenously, intraperitoneally or intralesionally. The treatment may be administered concurrently with other therapeutic treatments such as cytokines, surgical excision of melanoma lesions and chemotherapeutic drugs.

### Example 5

### Treatment of Patients with Metastatic Melanoma

In this protocol, patients with advanced melanoma are immunized with an antigenic cancer epitope.

Patients eligible for the trial must have evidence of measurable or evaluable metastatic melanoma that has failed standard effective therapy. Patients must have tumors that express the CAG-3 antigen as evidenced by PCR or Northern Blot analysis of tumor cell RNA.

patients receive either 1ng, 1µg, 1mg or 500mg/kg body weight of a cancer peptide (SEQ. ID NO: 4, 5, 14, 25, 34-38, 41, 42, 46 or 47) intravenously at day zero, day 7 and day 14 alone or in combination with IL2 and/or an immunostimulatory molecule. Patients are evaluated for toxicity, immunologic effects and therapeutic efficacy.

Lymphocytes taken from the treated patients are tested for specific response to the CAG-3 cancer antigen (SEQ. ID NO: 4, 5, 14, 25, 34-38, 41, 42, 46 or 47).

A complete response is defined as the disappearance of all clinical evidence of disease that lasts at least four weeks. A partial response is a 50% or greater decrease in the sum of the products of the perpendicular diameter of all measurable lesions for at least four weeks with no appearance of new lesions or increase in any lesions. Minor responses are defined as 25-49% decrease in the sum of the products of the perpendicular diameters of all measurable lesions with no appearance of new lesions and no increase in any lesions. Any patient with less than a partial response is considered a non-responder. The appearance of new lesions or greater than 25% increase in the product of perpendicular diameters of prior lesions following a partial or complete response is considered as a relapse.

### Example 6

### Recognition of CAG-3 antigens on tumor cells by CTL clones

In previous studies, we have isolated a number ofT cell clones from the TIL586 bulk cell line by the limiting dilution method (Wang et al. 1996b, J. Exp. Med. 183:1131-1140). Several clones recognized TRP-1 or TRP-2. However, two T cell clones isolated from the same TIL586 cell line recognized neither TRP-1 or TRP-2 but were capable of recognizing 586mel as well as HLA-A31+ melanocytes (Fig. 1A through 1C). These results suggested that these T cell clones recognized additional tumor antigens on the 586mel tumor cells. These T cell clones were then expanded and CTL clones 5 and 10 were used to screen cDNA libraries.

### Example 7

### Identification of a cDNA encoding a tumor antigen recognized by T cell clones

To determine the HLA molecule responsible for presenting antigen to CTL clones 5 and 10, we transfected HLA-A31 cDNA into A31-negative tumor lines 397mel and 624mel and tested for recognition by the CTL clone. Transfectants of 397mel and 624mel expressing HLA-A31 were significantly recognized by CTL clones 5 and 10 (Table 2).

**Table 2**

| **Specific secretion of GM-CSF by CTL clones 5 and 10 is HLA-A31-restricted** | | | | |
|---|---|---|---|---|
| Stimulators | | | GM-CSF secretion (pg/ml) | |
| Cell lines | Transfected gene | HLA-A31 expression | clone 5 | clone 10 |
| None | None | - | <50 | <50 |
| 397mel | None | - | <50 | <50 |
| 397mel | HLA-A31 | + | 3840 | 4280 |
| 624mel | None | - | <50 | <50 |
| 624mel | HLA-A31 | + | 4650 | 4320 |
| 586mel | None | + | 4050 | 3900 |
| 586EBVB | None | + | <50 | <50 |

| | | | | |
|---|---|---|---|---|
| GM-CSF in the supernatant was measured after 24 h incubation of 5 x 10⁴ CTL clone 5 or 10 cells with melanoma cell lines or media alone. | | | | |

Since only a limited number of T cells were available, we first tested whether or not these T cells recognized previously identified tumor antigens or melanocyte-lineage differentiation proteins. Recognition of COS-7 cells transfected with HLA-A31 cDNA and genes encoding the known tumor antigens or putative antigens including MART-1 (Kawakami et al. 1994a, Proc. Natl. Acad. Sci USA 91:3515-3519), gp75 (Wang et al. 1995, J. Exp. Med. 181:799-804), gplOO (Kawakami et al. 1994b, Proc. Natl. Acad. Sci. USA 91 :6458-6462), tyrosinase (Brichard et al. 1993, J. Exp. Med. 178:489-495), and TRP-2 (Yokoyama et al. 1994, Biochim. Biophys. Acta 1217:317-321; Bouchard et al. 1994, Eur. J. Biochem. 219:127-134) by CTL clone 5 or 10 was tested. COS cells transfected with HLA-A31 alone, TRP-1 or TRP-2 alone did not confer recognition by the T cell clones (Figure 2). Moreover, COS cells transfected with HLA-A31 and other tumor genes failed to stimulate GM-CSF release from T cells, indicating that the T cell clone 5 or 10 did not recognize these tumor antigens, but recognized a new tumor antigen in the context of HLA-A31.

### Example 8

### Cloning of CAG-3 Gene

To identify the new tumor Ag(s) recognized by CTL clones 5 and 10, we made a cDNA library derived from 586mel. Each cDNA pool consisted of ~100 cDNA clones. After screening a total of 2.5 x 10⁵ cDNA clones, we identified 15 positive cDNA pools that conferred T cell recognition by CTL clone 5 or 10 when cotransfected into COS-7 along with HLA-A31 cDNA. The positive clones were then tested for recognition by both CTL clones 5 and 10. It was found that both CTL clones recognized the same cDNA pools. Individual colonies were isolated from each positive pool and tested for T cell reactivity. Representative data are shown in Fig. 4. CTL clone 5 recognized COS-7 cotransfected with cDNA clone 1 or 2 and HLA-A31, but not COS-7 alone, COS-7 transfected with cDNA clone 1 or 2, or transfected only with the HLA-A31 cDNA (Fig. 4). DNA sequencing analysis indicated that all 10 cDNA positive clones from different positive pools overlapped, and the DNA and the amino acid sequence of these clones is shown in Fig. 3A. A search of all available databases revealed that the coding region of this gene, we named cancer antigen gene 3 (CAG-3, Genbank Accession Number AF038567), was identical to NY-ESO-1 which was recently reported to be an Ag recognized by a serum Ab derived from a patient with esophageal cancer (29). Our longest cDNA clone contained additional 37 nucleotides upstream of the previously reported 5'-end untranslated region. Two other proteins in the databases were found to contain homologous sequences in a limited region. The gene product of NY-ESO-1 (we have used NY-ESO-1 for CAG-3 in the following text) has 52% similarity to the tegument protein (UL36) of herpes simplex virus type 1 in the 64-amino acid segment and 47% similarity to enterobactin synthetase component F (serine-activating enzyme) in the 48-amino acid region (Fig. 3B).

### Example 9

### Breast Cancer Cells Recognized by CTL Clones

Northern blot analyses were performed using *NY-ESO-1* cDNA as a probe to evaluate the expression pattern in different tissues. Testis tissue was shown to be the only positive in the expression of NY-ESO-1 among the normal human tissues tested. NY-ESO-1 was found to be expressed in several types of cancers including melanoma and breast cancer (data not shown). These results were in consistent with data previously reported (29). To determine whether the melanoma reactive CTLs would also recognize other tumor cells, we tested T cell reactivity against HLA-A31 positive breast and prostate tumor cells by CTL clone 5. We used IFN-γ to monitor T cell recognition in these experiments because prostate tumor cells alone secrete GM-CSF, but not IFN-γ. As shown in Table 3, CTL clone 5 was capable of recognizing HLA-A31 positive 1295Br and 1315Br fresh breast tumor cells, but neither HLA-A31 negative 1405Br and 1411Br fresh breast tumor cells, nor HLA-A31 positive 1295 fibroblast cells derived from the autologous patient 1295. In addition, CTL clone 5 recognized the cultured HLA-A31 positive 1315Br (culture A and B) cells (Table 3), but did not respond to the cultured HLA-A31 negative cultured 1386Br breast cancer cells, nor the cultured HLA-A31 positive 1510 fibroblast. Although CTL clone 5 somehow did not respond to the cultured HLA-A31 positive 1315Br (culture A and B) cells in experiment 1, additional experiments showed T cell recognition of the cultured HLA-A31 positive 1315 Br (culture A and B) cells (data not shown). The expression of HLA-A31 and NY-ESO-1 in the fresh 1315Br and 1295Br tumor cells was confirmed by FACS analysis and RT-PCR analysis (data now shown). Expression of NY-ESO-1 was detected by RT-PCR using primers ESO-P2 and ESO-P5 in both 1295 and 1315 fresh breast tumor cells. CTL clone 5 recognized neither the HLA-A31⁺ 1535 prostate tumor cells because of lack of expression of NY-ESO-1 nor the HLA-A31-1542 prostate tumor cells (Table 3). These results strongly suggested that an antigenic peptide of NY-ESO-1 was expressed at sufficient levels on the surface of breast tumor cells to be recognized by T cells. Therefore, NY-ESO-1 may serve as an immune target for the immunotherapy of patients with breast cancer. NY-ESO-1 is also expressed in a high percentage (about 67%) of small cell lung carcinoma (data not shown).

**Table 3**

| **Recognition of breast tumor cells by CTL clone 5^{a}** | | | |
|---|---|---|---|
| Stimulators | | IFNγ release (pg/ml) by CTL clone 5 | |
| Target cells | HLA-A31 expression | Expt. 1 | Expt. 2 |
| 586mel | + | 225 | 488 |
| 397mel | - | 7 | 35 |
| 1315Br (fresh tumor #1) | + | 171 | 520 |
| 1315Br (fresh tumor #2) | + | 106 | 126 |
| 1295Br (fresh tumor) | + | 265 | 358 |
| 1295 Fibroblast | + | 4 | 24 |
| 1405 Br (fresh tumor) | - | 15 | 28 |
| 1411Br (fresh tumor) | - | ND | 74 |
| 1315Br (culture A) | + | 23 | 224 |
| 1315Br (culture B) | + | 0 | 155 |
| 1398Br | - | 0 | 36 |
| 1510 Fibroblast | + | 8 | ND |
| 1535 Prostate | + | 24 | ND |
| 1542 Prostate | - | 17 | ND |

| | | | |
|---|---|---|---|
| ^{a}FN-γ in the supernatant was measured after an 18-h incubation of 1 x 10⁵ CTL clone 5. In experiments 1 and 2 cytokine release from stimulators alone was <10 pg/ml. | | | |

### Example 10

### The peptide epitopes recognized by CTL clone 5

To screen for epitopes from the coding region of CAG-3 DNA an HLA peptide motif search was performed. These results are shown in Table 4 and Table 5.

**Table 4**

| **HLA peptide motif search results** | |
|---|---|
| **User Parameters and Scoring Information** | |
| Method selected to limit number of results | explicit number |
| Number of results requested | 30 |
| HLA molecule type selected | A-3101 |
| Length selected for subsequences to be scored | 10 |
| Echoing mode selected for input sequence | Y |
| Echoing format | numbered lines |
| Length of user's input peptide sequence | 180 |
| Number subsequence scores calculated | 171 |
| Number of top-scoring subsequences reported back in scoring output table | 30 |

| **Scoring Results** | | | |
|---|---|---|---|
| Rank | Start Position | Subsequence Residue Listing | Score (Estimate of Half Time of Disassociation of a Molecule Containing This Subsequence) |
| 1 | 127 | TVSGnILTIR | 4.000 Seq. ID No. 84 |
| 2 | 134 | TIRLtAADHR | 2.000 Seq. ID No. 85 |
| 3 | 97 | ATPMeAELAR | 2.000 |
| 4 | 170 | FLAQpPSGQR | 2.000 |
| 5 | 98 | TPMEaELARR | 1.200 |
| 6 | 77 | RCGArGPESR | 0.600 |
| 7 | 68 | AASG1NGCCR | 0.200 |
| 8 | 171 | LAQPpSGQRR | 0.200 |
| 9 | 163 | TQCFIPVFLA | 0.120 Seq. ID No. 86 |
| 10 | 153 | LQQLsLLMWI | 0.080 Seq. ID No. 87 |
| 11 | 115 | PLPVpGVLLK | 0.080 Seq. ID No. 88 |
| 12 | 152 | CLQQ1SLLMW | 0.080 Seq. ID No. 89 |
| 13 | 131 | NILTiRLTAA | 0.080 Seq. ID No. 90 |
| 14 | 126 | FTVSgNILTI | 0.080 Seq. ID No. 91 |
| 15 | 43 | RGPRgAGAAR | 0.060 |
| 16 | 158 | LLMWiTQCFL | 0.060 Seq. ID No. 92 |
| 17 | 87 | LLEFyLAMPF | 0.040 Seq. ID No. 93 |
| 18 | 161 | WITQcFLPVF | 0.040 Seq. ID No. 94 |
| 19 | 157 | SLLMwITQCF | 0.040 Seq. ID No. 95 |
| 20 | 93 | AMPFaTPMEA | 0.040 Seq. ID No. 96 |
| 21 | 72 | LNGCcRCGAR | 0.040 |
| 22 | 154 | QQLS1LMWIT | 0.040 Seq. ID No. 97 |
| 23 | 86 | RLLEfYLAMP | 0.024 Seq. ID No. 98 |
| 24 | 143 | RQLQ1SISSC | 0.024 Seq. ID No. 99 |
| 25 | 71 | GLNGcCRCGA | 0.020 Seq. ID No. 100 |
| 26 | 91 | YLAMpFATPM | 0.020 Seq. ID No. 101 |
| 27 | 22 | GIPDgPGGNA | 0.020 Seq. ID No. 102 |
| 28 | 53 | ASGPgGGAPR | 0.020 |
| 29 | 144 | QLQLsISSCL | 0.020 Seq. ID No. 103 |
| 30 | 133 | LTIR1TAADH | 0.020 Seq. ID No. 104 |

**Table 5**

| **HLA peptide motif search results** | |
|---|---|
| **User Parameters and Scoring Information** | |
| Method selected to limit number of results | explicit number |
| Number of results requested | 30 |
| HLA molecule type selected | A-3101 |
| Length selected for subsequences to be scored | 9 |
| Echoing mode selected for input sequence | Y |
| Echoing format | numbered lines |
| Length of user's input peptide sequence | 181 |
| Number subsequence scores calculated | 173 |
| Number of top-scoring subsequences reported back in scoring output table | 30 |

| **Scoring Results** | | | |
|---|---|---|---|
| Rank | Start Position | Subsequence Residue Listing | Score (Estimate of Half Time of Disassociation of a Molecule Containing This Subsequence) |
| 1 | 172 | AQPPSGQRR | 2.000 |
| 2 | 98 | TPMBAELAR | 1.200 |
| 3 | 99 | PMEAELARR | 0.400 |
| 4 | 86 | RLLEFYLAM | 0.240 Seq. ID No. 63 |
| 5 | 38 | GATGGRGPR | 0.200 |
| 6 | 44 | GPRGAGAAR | 0.200 |
| 7 | 171 | LAQPPSGQR | 0.200 |
| 8 | 154 | QQLSLLMWI | 0.160 Seq. ID No. 64 |
| 9 | 116 | LPVPGVLLK | 0.160 Seq. ID No. 65 |
| 10 | 120 | GVLLKEFTV | 0.120 Seq. ID No. 66 |
| 11 | 131 | NILTIRLTA | 0.080 Seq. ID No. 67 |
| 12 | 161 | WITQCFLPV | 0.080 Seq. ED No. 68 |
| 13 | 127 | TVSGNILTI | 0.080 Seq. ID No. 69 |
| 14 | 153 | LQQLSLLMW | 0.080 Seq. ID No. 70 |
| 15 | 159 | LMWITQCFL | 0.060 Seq. ID No. 71 |
| 16 | 158 | LLMWITQCF | 0.060 Seq. ID No. 72 |
| 17 | 132 | ILTIRLTAA | 0.040 Seq. ID No. 73 |
| 18 | 148 | SISSCLQQL | 0.040 Seq. ID No. 74 |
| 19 | 128 | VSGNILTTR | 0.040 |
| 20 | 145 | LQLSISSCL | 0.040 Seq. ID No. 75 |
| 21 | 135 | IRLTAADHR | 0.040 |
| 22 | 152 | CLQQLSLLM | 0.040 Seq. ID No. 76 |
| 23 | 110 | AQDAPPLPV | 0.040 Seq. ID No. 77 |
| 24 | 164 | QCFLPVFLA | 0.036 Seq. ID No. 78 |
| 25 | 143 | RQLQLSISS | 0.024 Seq. ID No. 79 |
| 26 | 108 | SLAQDAPPL | 0.020 Seq. ID No. 80 |
| 27 | 73 | NGCCRCGAR | 0.020 Seq. ID No. 81 |
| 28 | 134 | TIRLTAADH | 0.020 Seg. ID No. 82 |
| 29 | 54 | SGPGGGAPR | 0.020 |
| 30 | 69 | ASGLNGCCR | 0.020 Seq. ID No. 83 |

Peptides were synthesized based on the peptide binding motif for HLA-A31 (hydrophobic residues at position 2 and positively charged residues at position 9) (Rammensee et al. 1995, Immunogenetics 41:178-228) and tested for reactivity with CTL clone 5.

These peptides were pulsed onto HLA-A31-positive 1510EBV B cells and tested for their ability to stimulate cytokine release by CTL clone 5. As shown in Table 6, the 10-mer peptide ESO10-53 (ASGPGGGAPR), starting at position 53 of the NY-ESO-1 protein was strongly recognized by CTL clone 5, while the overlapping 9-mer peptides, ESO9-54 as well as ESO10-127, were weakly recognized when pulsed onto 1510EVB B cells. CTL clone 10 recognized the same peptide as CTL clone 5 (data not shown). Interestingly, CTL clone 2 did not recognize any of these peptides (Table 6), even though it recognized 586mel and COS-7 transfected with NY-ESO-1 (see below). The reactivity of CTL clone 5 was undetectable when either the ESO9-54 or the ESO10-127 peptides were used at concentrations below 100 nM to sensitize EBV cells.

**Table 6**

| **Screening of synthetic peptides with reactivity to CTL Clone 5^{a}** | | |
|---|---|---|
| **Target cells pulsed with peptide** | | **CTL clone 5** |
| **from NY-ESO-1** | | **GM-CSF release (pg/ml)** |
| **Peptides from CAG-3** | | |
| 1510EBV + ES09-90 | AQPPSGQRR | <50 (SEQ. ID NO: 6) |
| 1510EBV + ESO9-98 | TPMEAELAR | <50 (SEQ. ID NO: 7) |
| 1510EBV + ESO9-99 | PMEAELARR | <50 (SEQ. ID NO: 8) |
| 1510EBV + ESO9-38 | GATGGRGPR | <50 (SEQ. ID NO: 9) |
| 1510EBV + ESO9-45 | GPRGAGAAR | <50 (SEQ. ID NO: 10) |
| 1510EBV + ESO9-89 | LAQPPSGQR | <50 (SEQ. ID NO: 11) |
| 1510EBV + ESO9-128 | VSGNILTIR | <50 (SEQ. ID NO: 12) |
| 1510EBV + ESO9-35 | IRLTAADHR | <50 (SEQ. m NO: 13) |
| 1510EBV - ESO9-54 | SGPGGGAPR | 304 (SEQ. ID NO: 14) |
| 1510EBV + ESO10-127 | TVSGNILTIR | 293 (SEQ. ID NO: 15) |
| 1510EBV + ESO10-34 | TIRLTAAOHR | <50 (SEQ. ID NO: 16) |
| 1510EBV + ESO10-91 | ATPNFEAELAR | <50 (SEQ. ID NO: 17) |
| 1510EBV + ESO10-170 | FLAQPPSGQR | <50 (SEQ. ID NO: 18) |
| 1510EBV + ESO10-98 | TPMEAELARR | <50 (SEQ. ID NO: 19) |
| 1510EBV + ESO10-77 | RCGARGPESR | <50 (SEQ. ID NO: 20) |
| 1510EBV + ESO10-68 | AASGLNGCCR | <50 (SEQ. ID NO: 21) |
| 1510EBV + ESO10-171 | LAQPPSGQRR | <50 (SEQ. ID NO: 22) |
| 1510EBV + ESO10-44 | RGPRGAGAAR | <50 (SEQ. ID NO: 23) |
| 1510EBV + ESO10-72 | LNGCCRCGAR | <50 (SEQ. ID NO: 24) |
| 1510EBV + ESO10-53 | ASGPGGGAPR | >2000 (SEQ. ID NO: 25) |
| 1510EBV+None | | <50 |

| | | |
|---|---|---|
| ^{a}1510 EBV cells were incubated with individual peptide at a concentration of 0.1 µg/ml for 90 min. GM-CSF release was measured after co-incubation of peptide-loaded 1510EBV cells with CTL clone 5. GM-CSF release was measured after coincubation of peptide-loaded 1510 EBV cells with CTL clone 5. 1510EBV was a EBV transformed B cell line expressing HLA-A31. | | |

### Example 11

### Recognition of Modified Peptides

To examine whether CTL clone 5 also recognized peptides that contained the core amino acid sequence with the extension of amino acid residues at either the N or C terminus, we made overlapping 11-mer, 12-mer, 13-mer, 14-mer, and 15-mer peptides, as well as several peptides containing substitutions at either position 1, 2 or 10 of ESO10-53 (Table 7 and Figure 3A). CTL clone 5 was capable of recognizing 11-mer, 12-mer, 13-mer, 14-mer, and 15-mer peptides with amino acid extensions at the N terminus of the ESO-53 core peptide, although the longer peptides appeared to stimulate significantly less GM-CSF secretion than did the ESO10-53 10-mer peptide (Table 7). However, an extension of only a single amino acid residue at the C terminus of ESO10-53 abrogated its ability to stimulate T cells (Table 7). CTL clone 5 did not recognize the 8-mer peptide.

Titration experiments demonstrated that CTL reactivity was detectable at 10 nM concentration of ESO10-53 peptide. GM-CSF release from CTL clone 5 increased with the increasing peptide concentrations, and no plateau was reached at 10 µM peptide concentration (Fig. 5A). In addition to measuring cytokine release stimulated by ESO10-53, the ability of this peptide to sensitize target cells for lysis by CTL clone 5 was examined. CTL clone 5 was capable of lysing HLA-A31-positive tumor 586 mel, but not the HLA-A31-negative and NY-ESO-1-positive tumor 397mel (Fig. 5B). CTL clone 5 lysed > 60% of either 586EBV or 1510EBV that had been incubated with the ESO10-53 peptide at an E:T ratio of 40:1, and - 5-10% lysis of target cells was observed at an E:T ratio of 2.5:1 E:T. CTL clone did not lyse either 586EBV or 1515EBV B cells alone or pulsed with an irrelevant peptide, nor did it lyse the HLA-A31-negative.T2 cells pulsed with the ESO10-53 peptide (Fig. 5C).

Next, it was tested whether T cell recognition of the 10-mer peptide could be improved by substituting amino acids at anchor residues. A number of synthetic peptides with modification at residues 1, 2 and 10 were made and tested for recognition by CTL clone 5 when pulsed onto 586EBV B cells (Table 7). The modified 10-mer peptides with a substitution at position 2 derived from the wild-type ASGPGGGAPR were still recognized by CTL clone 5 when pulsed on 586EBV B cells. The reactivity of peptides containing a substitution of either Ala, Ile, Leu or Val at position 2 was lower than that of the wild-type peptide, while one peptide containing a substitution of Thr for Ser at position 2 resulted in a slightly higher reactivity than the wild-type ESO10-53 peptide. In contrast, peptides containing substitutions of Arg with Lys or His completely lost their ability to stimulate T cells, suggesting that the Arg at the C-terminus of the ESO10-53 peptide represents a critical anchor residue. Peptides with a substitution at position 1 were recognized poorly or not recognized at all by CTL clone 5 (Table 7). These results indicate that the ESO-53 peptide, ASGPGGGAPR, represents the best peptide for T cell recognition.

### Example 12

Antigenic Peptides Derived From An Alternative Open Reading Frame Two additional CTL clones, clones 2 and 14, appeared to recognize 586mel as well as COS-7 cells transfected with NY-ESO-1 and HLA-A31 cDNA, but failed to recognize the ESO 10-53 peptide (Fig. 6A-6H). CTL clone 5 and TTL1244 were used for the specificity controls. Additional experiments showed that CTL clone 2 did not respond to any of 19 other peptides containing the HLA-A31 binding motif derived from the normal open reading frame of NY-ESO-1 (Table 6). To test the hypothesis that CTL may recognize a peptide from a gene product translated from an alternative open reading frame of the same gene, synthetic peptides were made with HLA-A31 binding motif on the basis of amino acid sequence predicted from the second open reading frame (ORF2) (Fig. 3A). Strikingly, CTL clone 2 recognized ESORF2-9-19 (AAQERRVPR) as well as the overlapping ESORF2-10-18 (LAAQERRVPR) peptides when pulsed onto 1510EBV B cells. Representative data for CTL clone 2 is shown in Fig. 7A. CTL clone 14 recognized the same peptides as CTL clone 2 (Data not shown). These results suggest that CTL clones 2 and 14 recognized an antigenic peptide derived from the ORF2 (Fig. 3A). A protein database search revealed that the 58 amino acid protein of ORF2 has a 52% similarity to the chain A of glutamate dehydrogenase in a 25 amino acid region (34). Peptide titration experiments demonstrated that CTL clone 2 was capable oflysing 1510EBV pulsed with ESORF2-10-18 (LAAQERRVPR) at relatively low concentrations of peptide, but failed to lyse 1510EBV pulsed with ESO10-53 or HLA-A31-negative 1102EBV pulsed with ESORF2-10-18 (Fig. 7B). In addition, CTL clone 2 also recognized overlapping 11-mer, 12-mer, and 13-mer peptides with amino acid extensions at the N terminus of the ESORF2-10-18 peptide at relatively high concentrations (data not shown).

Additional experiments were carried out to determine whether CTL clones recognize the ORF2 gene product of the NY-ESO-1 in other tumor types. As shown in Fig. 7C, the recognition pattern of CTL clone 2 was similar to that of CTL: clone 5 on tumor cells. CTL clone 2 recognized HLA-A31 positive fresh 1315Br and 1295Br breast tumors as well as 586mel and 1388mel, but did not recognize HLA-A31 negative fresh 1411Br breast tumor, 397mel, nor the HLA-A31 negative 1295 fibroblast. Although 1353mel expresses HLA-A31, neither CTL clone 2 nor clone 5 responded to 1353mel because 1353mel is NY-ESO-1 negative tumor. As previously demonstrated, CTL clone 5 recognized the ESO10-53 ASGPGGGAPR peptide and CTL clone 2 recognized the ORF2-10-18 LAAQERRVPR peptide derived from the ORF2 following incubation with 1510EBV B cells (Fig. 7C). These results strongly suggest that the ORF2 gene product was translated, processed and presented in melanoma as well as breast tumors. Therefore, *NY-ESO-1* encodes two different proteins: a protein with 180 amino acids (ORF1) recognized by CTL clones 5 and 10, and a protein with 58 amino acids (ORF2) recognized by CTL clones 2 and 14.

**Table 7**

| **Recognition of the modified peptides by CTL clone 5^{a}** | | | |
|---|---|---|---|
| Target cells pulsed with peptides | | GM-CSF release by CTL clone 5 | |
| | | EXPT. 1 | EXPT. 2 |
| | | (1510EBV) | (586EBV) |
| 1510EBV + | AGAARASGPGGGAPR | 728 | 532 (SEQ. ID NO: 26) |
| 1510EBV + | GAARASGPGGGAPR | 659 | 678 (SEQ. ID NO: 27) |
| 1510EBV + | AARASGPGGGAPR | 844 | 738 (SEQ. ID NO: 28) |
| 1510EBV + | ARASGPGGGAPR | 840 | 738 (SEQ. ID NO: 29) |
| 1510EBV + | RASGPGGGAPR | 851 | 708 (SBQ. ID NO: 30) |
| 1510EBV + | SGPGGGAPR | <50 | <50 (SEQ. ID NO: 14) |
| 1510EBV + | GPGGGAPR | <50 | <50 (SEQ. ID NO: 31) |
| 1510EBV + | ASGPGGGAPRG | <50 | <50 (SEQ. ID NO: 32) |
| 1510EBV + | SGPGGGAPRG | <50 | <50 (SEQ. ID NO: 33) |
| 1510EBV + | ASGPGGGAPR | 1097 | 1044 (SEQ. ID NO: 25) |
| 1510EBV + | AAGPGGGAPR | 688 | 676 (SEQ. ID NO: 34) |
| 1510EBV + | AIGPGGGAPR | 428 | 550 (SEQ. ID NO: 35) |
| 1510EBV + | ALGPGGGAPR | 200 | 378 (SEQ. ID NO: 36) |
| 1510EBV + | AVGPGGGAPR | 556 | 512 (SEQ. ID NO: 37) |
| 1510EBV + | ATGPGGGAPR | 1630 | 1158 (SEQ. ID NO: 38) |
| 1510EBV + | ASGPGGGAPK | <50 | <50 (SEQ. ID NO: 39) |
| 1510EBV + | ASGPGGGAPH | <50 | <50 (SEQ. ID NO: 40) |
| 1510EBV + | TSGPGGGAPR | 320 | 346 (SEQ. ID NO: 41) |
| 1510EBV + | VSGPGGGAPR | 825 | 666 (SEQ. ID NO: 42) |
| 1510EBV + | LSGPGGGAPR | <50 | <50 (SEQ. ID NO: 43) |
| 1510EBV + | RSGPGGGAPR | <50 | <50 (SEQ. ID NO: 44) |
| 1510EBV + | None | <50 | <50 |

| | | | |
|---|---|---|---|
| ^{a}586EBV or 1510EBV cells were incubated with individual peptide at a concentration of 0.1µg/ml for 90 min. GM-CSF release was measured after coincubation of peptide-loaded EBV cells with 5 x 10⁴ CTL clone 5. The wild-type 10-mer ESO10-53 peptide is underlined. Peptides with amino acid substitutions are marked by bold face and underscored. 586EBV and 1510EBV were HLA-A31-positive EBV transformed B cell lines. | | | |

### DISCUSSION

### NY-ESO-1 Is A Cellular Immune Target

Five differentiation antigens including tyrosinase, MART-1, gp 100, TRP-l/gp75 and TRP-2 have been identified as melanoma Ags recognized by T cells derived from TILs (13-19), which have been shown to be associated with antitumor reactivity in vivo. Thus far, no TILs available in the Surgery Branch at National Cancer Institute recognize MAGE-1, BAGE and GAGE, which are expressed only in testis and cancer cells. Here we show that NY-ESO-1, another cancer-shared Ag, is a tumor Ag recognized by HLA-A31-restricted T cells. NY-ESO-1 was independently isolated using the autologous serum from a patient with esophageal cancer (29), suggesting that the NY-ESO-1 gene product was an immune target for Ab-mediated immunity. Results in this study provide evidence that NY-ESO-1 is also an immune target recognized by T cells. This is further supported by a recent report that HLA-A2 restricted CTL recognize NY-ESO-1 established in a melanoma patient (35). Several tumor antigens, including MAGE-1, tyrosinase, TRP-1, recognized by CTL, have been found to be reactive with antibody as well (36, 37). Since NY-ESO-1 is not expressed in normal human tissues except the testis, which does not express MHC class I molecules and is considered as an immunologically privileged site, this gene product may consistitute a safe immune target for the immunotherapy of patients with cancer. NY-ESO-1 Is A Breast Cancer Ag

Based on its gene expression pattern, NY-ESO-1 belongs to a member of an expanding family of antigens (Ags) including MAGE-1, MAGE-3, BAGE, GAGE and HOM-MEL-40 (9-12,30). However, NY-ESO-1 is highly expressed in a significant proportion of breast, prostate and bladder cancers (29) compared to MAGE, BAGE and GAGE. More importantly, CTL clones recognized two HLA-A31-positive fresh and cultured breast cancer cells (Table 2 and Fig. 7C). To our knowledge, this is the first demonstration of CTL recognition of NY-ESO-1 positive breast cancer cells. Although the expression of MAGE-1, MAGE-3 and others were reported to be detected by RT-PCR in breast tumors at a low frequency (<5-10%), CTL recognition of these breast tumors by the Ag-specific CTL has not been documented. It has been difficult to generate breast-reactive CTLs from PBL *in vitro* although MHC-restricted T cells that recognized HER-2/neu peptides on breast cancer cells have been reported (26-28). Identification of NY-ESO-1 peptides presented on the cell surface of breast cancers is important for the development of Ag-specific cancer vaccines for the treatment of patients with breast cancer. The CTL cloning approach described here represents a strategy for the isolation breast cancer Ags. Translation of Different ORFs As A Mechanism For Generating T Cell Epitopes

To define antigenic peptides, we found the 10-mer ASGPGGGAPR derived from the NY-ESO-1 protein as the best antigenic peptide recognized by CTL clone 5 although the 9-mer, 11-mer, 12-mer, 13-mer, 14-mer and 15-mer peptides were also recognized. This reactivity may be due to the presence of two proline residues in these peptides. Proline residues in the core peptide sequence may allow the peptides to bulge out of the MHC binding pocket, thus the anchored residues in the longer peptides can still fit into the HLA-A31 molecule. An alternative explanation is that the longer peptides may be processed to the shorter peptides by extracellular or serum proteases (38). However, when the longer peptides were pulsed onto 586EBV B cells in serum-free conditions, they were still recognized by CTL clone 5 (data not shown). This experiment, however, does not exclude the possibility that these longer peptides were processed by extracellular proteases. The modified peptide, ATGPGGGAPR, with a substitution of Thr for Ser appeared to slightly improve T cell recognition. It has been shown that modified peptides could improve binding affinity to the MHC class I molecule, thus enhancing the immunogenicity of peptides (39). It is not clear why CTL clone 5 recognized the unrelated ESO10-127 peptide, but its recognition was weak and only could be detected at a relatively high peptide concentration.

Interestingly, two additional CTL clones recognized COS-7 transfected with NY-ESO-1 plus HLA-A31 cDNA, but not the ESO10-53 peptide which was derived from the ORF1 of the NY-ESO-1 gene (Fig. 6A-6D). Further analysis showed that CTL clone 2 recognized a peptide from the gene product translated from an alternative open reading flame (ORF2) of NY-ESO-1. Although there are several examples that two different proteins are translated from different ORFs from a single viral mRNA (40), very few cases have been reported in single eukaryotic mRNA. Two examples have been reported: TRP-1/gp75 which encodes two different proteins, gp75 recognized by sera from a patient with melanoma and a 24 amino acid gene product recognized by CTLs (24, 37); and 43 kDa-Ag recognized by squarnous cell carcinoma reactive CTL (41). Interestingly, the cyclin-dependent kinase inhibitor p16 gene also encodes two gene products, p16 and p19*^{ARF}*. However, the alternative ORF gene product p19*^{ARF}* is translated from an alternatively spliced transcript and shares an identical sequence with the p16 transcript except the first exon (42). Recent studies showed that the alternative reading frame product p19*^{ARF}* plays a role in cell cycle regulation and tumor suppression (43). The NY-ESO-1 gene reported here encodes two different proteins: a 180 amino acid protein encoded by ORF1 and a 58 amino acid protein encoded by ORF2. The ORF2 product is located inside ORF1. Strikingly, CTL clones 5 and 10 recognized antigenic peptides derived from ORF1, while CTL clone 2 and 14 recognized an antigenic peptide from ORF2. Both CTL clones 2 and 5 recognized several HLA-A31⁺ melanomas as well as fresh breast tumors (Table 3 and Fig. 7C), suggesting that the translation of the alternative ORF2 may serve as a general mechanism *in vivo* for generating T cell epitopes.

The mechanism by which the alternative ORF is translated is currently not known. However, there are several potential explanations for the production of alternative reading frames. The leaky-scanning model is one possible explanation: ribosomes occasionally bypass the first AUG with a poor KOZAK consensus sequence and initiates translation at a downstream AUG (43, 44). In the case of NY-ESO-1, the start codon for translation of ORF 1 is not in the optimal context. There are four potential start codons that could be used to translate the ORF2. In our previous report, a CTL epitope was translated from an alternative reading frame of gp75/TRP-1 (24). Recognition of the epitope of ORF3 by CTL was affected by the presence of the first ATG codon used for the translation of the gp75 protein and was completely abolished when the internal ATG preceeding the T cell eptiope (ORF3P) was changed to ATC, suggesting that ribosomal scanning may be a possible mechanism. In the studies of the influenza nucleoprotein, CTL epitopes were produced via a ribosomal scanning mechanism (44). However, in a similar study, ribosomal frameshifting was suggested as a mechanism for the production of T cell epitopes (45). Other mechanisms have also been proposed for the production ofT cell epitopes derived from different genes. For example, several T cell epitopes were identified from gene products translated from a cryptic initiation codon of the 5' untranslated region (46,47), from a non-ATG-defined open reading frame (48), and from introns of transcript (22,49,50). Since both the alternative open reading frame of *gp75*/*TRP-1* and *NY-ESO-1* are located within the primary open reading frame, it is of particular interest to understand the underlying mechanism.

Although there are only a few examples of the usage of the alternative open reading frames in eukaryotes reported in the literature, we believe that more examples will be reported in the future when tumor-reactive CTL and autoantibodies are available and used to identify target proteins or peptides. Therefore, it is important to understand the biologic significance of the gene products translated from alternative open reading frames. One possibility is that these gene products serve as antigenic targets of the Ag processing machinery to increase the efficiency and capacity of the immune surveillance. Identification ofT cell epitopes derived from different open reading frames of NY-ESO-1 suggests that the identity of immunogenic peptides for cancer vaccines may not be limited only to peptides derived from the primary open reading frame. It is not clear at the present time whether the ORF2 gene product of NY-ESO-1 and the ORF3 gene product of gp75/TRP-1 have biologic functions in addition to immune responses ofT cells.

### References

1. Rosenberg, S.A., B.S. Packard, P.M. Aebersold, D. Solomon, S.L. Topalian, S.T. Toy, P. Simon, M.T. Lotze, J.C. Yang, C.A. Seipp, et al, 1998. Use of tumor infiltrating lymphocytes and interleukin-2 in the immunotherapy of patients with metastatic melanoma. Preliminary report. N. Engl. J. Med. 319:1676.
2. Rosenberg, S.A., J.Y. Yannelli and J.C. Yang, 1994. Treatment of Patients with metastatic melanoma using autologous tumor-infiltrating lymphocytes and interleukin-2. J. Natl. Cancer Inst. 86:1159.
3. Boon, T., J.C. Cerottini, B. Van den Eynde, P. van der Bruggen and A. Van Pel. 1994. Tumor antigens recognized by T lymphocytes. Annu. Rev. Immunol. 12:337.
4. Houghton, A.N. 1994. Commentary: cancer antigens: immune recognition of self and altered self. J. Exp. Med. 180:1.
5. Tsomides, T.J. and H.N. Eisen, 1994. Commentary: T-cell antigens in cancer. Proc. Natl. Acad. Sci. USA 91:3487.
6. Pardoll, D.M. 1994. News and views: a new look for the 1990s. Nature 369:357.
7. Wang, R.F., and S.A. Rosenberg, 1996. Human tumor antigens recognized by T lymphocytes: implications for cancer therapy. J. Leukocyte Biol. 60:296.
8. Rosenberg, S.A. 1997. Cancer vaccines based on the identification of genes encoding cancer regression antigens. Immunol. Today 18:175.
9. van der Bruggen, P., C. Traversari, PI: Chomez, C. Lurquin, E. Deplaen, B. Van den Eynde, A. Knuth and T. Boon, 1991. A gene encoding an antigen recognized by cytolytic T lymphocytes on a human melanoma. Science 254:1643.
10. Gaugler, B., B. Van den Eynde, P. van der Bruggen, P. Romero, J.J. Gaforio, E. De Plaen, B. Lethe, F. Brasseur and T. Boon, 1994. Human gene MAGE-3 codes for an antigen recognized on a melanoma by autologous cytolytic T lymphocytes. J. Exp. Med. 179:921.
11. Van Den Eynde, B., O. Peeters, O. De Backer, B. Gaugler, S. Lucas and T. Boon, 1995. A new family of genes coding for an antigen recognized by autologous cytolytic T lymphocytes on a human melanoma. J. Exp. Med. 182:689.
12. Boël P., C. Wildmann, M.L. Sensi, R. Brasseur, J.C. Renauld, P. Coulie, T. Boon and P. van der Bruggen, 1995. BAGE: a new gene encoding an antigen recognized on human melanomas by cytolytic T lymphocytes. Immunity 2:167.
13. Kawakami, Y., S. Eliyahu, C.H. Delgaldo, P.F. Robbins, L. Rivoltni, S.L. Topalian, T. Miki and S.A. Rosenberg, 1994. Cloning of the gene coding for a shared human melanoma antigen recognized by autologous T cells infiltrating into tumor. Proc. Natl. Acad. Sci. USA 91:3515.
14. Coulie, P.G., V. Brichard, A. Van Pel. T. Wolfe, J. Schneider, C. Traversari, S. Mattei, E.D. De Plaen, C. Lurquin, J.P. Szikora, J.C. Reauld and T. Boon, 1994. A new gene coding for a differentiation antigen recognized by autologous cytolytic T lymphocytes on HLA-A2 melanomas. J. Exp. Med. 180:35.
15. Kawakami, Y., S. Eliyahu, C.H. Delgado, P.F. Robins, K. Sakaguchi, E. Appella, J.R. Yannelli, G.J. Adema, T. Miki and S.A. Rosenberg, 1994. Identification of a human melanoma antigen recognized by tumor infiltrating lymphocytes associated with in vivo tumor rejection. Proc. Natl. Acad. Sci. USA 91:6458.
16. Brichard, V., A. Van Pel, T. Wölfel, E. De Plaen, B. Lethë, P. Coulie and T. Boon, 1993. The tyrosinase gene codes for an antigen recognized by autologous cytolytic T lymphocytes on HLA-A2 melanomas. J. Exp. Med. 178:489.
17. Robbins, P.F., M. El-Gamil, Y. Kawakami, E. Stevens, J. Yannelli and S.A. Rosenberg, 1994. Recognition of tyrosinase by tumor infiltrating lymphocytes from a patient responding to immunotherapy. Cancer Res. 54:3124.
18. Wang, R.F., P.F. Robbins, Y. Kawakami, X.Q. Kang and S.A. Rosenberg, 1995. Identification of a gene encoding a melanoma tumor antigen recognized by HLA-A31-restricted tumor-infiltrating lymphocytes. J. Exp. Med. 181:799.
19. Wang, R.F., E. Appella, Y. Kawakami, X.Q. Kang and S.A. Rosenberg, 1995. Identification of TRP-2 as a human tumor antigen recognized by cytotoxic T lymphocytes. J. Exp. Med. 184:2207.
20. Wolfel, T., M. Hauer, J. Schneider, M. Serrano, C. Wolfel, E. Klehmann-Hieb, E. De Plaen, T. Hankeln, K.H. Meyer Zum Buschenfeld and D. Beach, 1995. A p16INK4a-insensitive CDK4 mutant targeted by cytolytic T lymphocytes in a human melanoma. Science 269:1281.
21. Robbins, P.F., M. El-Gamil, Y.F. Li, Y. Kawakami, D. Loftus, E. Appella and S.A. Rosenberg, 1996. A mutated β-catenin gene encodes a melanoma-specific antigen recognized by tumor infiltrating lymphocytes. J. Exp. Med. 183:1185.
22. Coulie, P.G., F. Lehmann, B. Lethe, J. Herman, C. Lurquin, M. Andrawiss and T. Boon, 1995. A mutated intron sequence codes for an antigenic peptide recognized by cytolytic T lymphocytes on a human melanoma. Proc. Natl. Acad. Sci. USA 92:7976.
23. Mandruzzato, S., F. Brasseur, G. Andry, T. Boon and P. van der Bruggen, 1997. A CASP-8 mutation recognized by cytolytic T lymphocytes on a human head and neck carcinoma. J. Exp. Med. 186:785.
24. Wang, R.F., M.R. Parkhurst, Y. Kawakami, P.F. Robbins and S.A. Rosenberg, 1996. Utilization of an alternative open reading frame of a normal gene in generating a novel human cancer antigen. J. Exp. Med. 183:1131.
25. Bloom, M.D., D. Perry-Lalley, P.F. Robbins, Y. Li, M. El-Gamil, S.A. Rosenberg and J.C. Yand, 1997. Identification of tyrosinase-related protein 2 as a tumor rejection antigen for the B16 melanoma. J. Exp. Med. 185:453.
26. Disis, M.L., J.W. Smith, A.E. Murphy, W. Chen and M.A. Cheever, 1994. In vitro generation of human cytotoxic T-cells specific for peptides derived from the HER-2/new protooncogene protein. Cancer Res. 54:1071.
27. Fisk. B., B. Chesak, J.S. Pollack, J.T. Wharton and C.G. Ioannides, 9114. Oligopeptide induction of a cytotoxic T lymphocyte response to HER-2/Neu proto-oncogene in vitro. Cell Immunol. 157:415.
28. Peoples, G.E., I. Yosino, C.C. Douville, J.V.R. Andrews, P.S. Goedegebuure and T.J. Eberlein, 1994. TCR Vβ3⁺ and Vβ6⁺ CTL recognized tumor-associated antigens related to HER-2/neu expression in HLA-A2⁺ ovarian cancers. J. Immunol. 152:4993.
29. Chen Y.-T., M.J. Scanlan, U. Sahin, O. Tureci, A.O. Gure, S. Tsang, B. Williamson, E. Stockert, M. Pfreundschuh and L.J. Old. 1997. A testicular antigen aberrantly expressed in human cancers detected by autologous antibody screening. Proc. Natl. Acad. Sci. USA 94:1914.
30. Topalian, S., D. Solomon, F.P. Avis, A.E. Chang, D.L. Freeksen, W.M. Linehan, M.T. Lotze, C.N. Robertson, C.A. Seipp, P. Simon, C.G. Simpson and S.A. Rosenberg, 1988. Immunotherapy of patients with advanced cancer using tumor infiltrating lymphocytes and recombinant interleukin-2: a pilot study, J. Clin. Oncol. 6:839.
31. Wang. R.F., S. Johnston, S. Southwood, A. Sette and S.A. Rosenberg, 1998.d Recognition of an antigenic peptide derived from TRP-2 by cytotoxic T lymphocytes in the context of HLA-A31 and -A33. J. Immunol. 160:890.
32. Riddell, S.R. and P.D. Greenberg, 1995. Principles for adoptive T cell therapy of human viral diseases. Annu. Rev. Immunol. 13:545.
33. Rammensee, H.G., T. Friede and S. Stevanoviic, 1995. MHC ligands and peptide motifs: first listing. Immmunogenetics 41:178.
34. Baker, P.J., K.L. Britton, P.C. Engel, G.W. Farrants, K.S. Lilley, D.W. Rice and T.J. Stillman, 1992. Subunit assembly and active site location in the structure of glutamate dehydrogenase. Proteins 12:75.
35. Jager, E., Y.-T. Chen, J.W. Drijfout, J. Karbach, M. Ringhoffer, D. Jager, M. Arand, H. Wada, Y. Noguchi, E. Stockert, LJ. Old and A. Knuth, 1998. Simultaneous humoral and cellular immune response against cancer-testis antigen NY-ESO-1: definition of human histocompatibility leukocyte antigen (HLA)-A2-binding peptide eptiopes. J. Exp. Med. 187:265.
36. Sahin, U., O. Tureci, H. Schmitt, B. Cochlovius, T. Johannes, R. Schmits, F. Stenner, G. Luo, I. Schobert and M. Pfreundschuh, 1995. Human neoplasms elicit multiple immune responses in the autologous host. Proc. Natl. Acad. Sci. USA 92:11810.
37. Mattes, J.J., T.M. Thomson, L.J. Old and K.O. Lloyd, 1983. A pigmentation-associated, differentiation antigen of human melanoma defined by a precipitating antibody in human serum. Int. J. Cancer 32:717.
38. Kozlowski, S., M. Corr, M. Shirai, L.F. Boyd, C.D. Pendleton, J.A. Berzofsky and D.H. Margulies, 1993. Multiple pathways are involved in the extracellular processing of MHC class I-restricted peptides. J. Immunol. 151:4033.
39. Parkhurst, M.R., M. Salgaller, S. Southwood, P. Robbins, A. Sette, S.A. Rosenberg and Y. Kawakami, 1996. Improved induction of melanoma reactive CTL with peptides from the melanoma antigen gp100 modified at HLA-A0201 binding residues. J. Immunol. 157:2539.
40. Fischer, F., D. Peng, S.T. Hingley, S.R. Weiss and P.S. Masters, 1997. The internal open reading frame within the nucleocapside gene of mouse hepatitis virus encodes a structural protein that is not essential for viral replication. J. Virol. 71:996.
41. Quelle, D.E., F. Zindy, R.A. Ashmun and C.J. Sherr, 1995. Alternative reading frames of the INK4a tumor suppressor gene encode two unrelated proteins capable of inducing cell cycle arrest. Cell 83:993.
42. Kamijo, T., F. Zindy, M.F. Roussel, D.E. Quelle, J.R. Downing, R.A. Ashmun, G. Grosveld and C.J. Sheer, 1997. Tumor suppression at the mouse INK4a locus mediated by the alternative reading frame product p19^{ARF}. Cell 91:649.
43. Kozak, M. 1994. Determinants of translational fidelity and efficiency in vertebrate mRNAs. Biochimie 76:815.
44. Bullock, T.N.J. and L.C. Eisenlohr, 1996. Ribosomal scanning past the primary initiation codon as a mechanism for expression of CTL epitopes encoded in alternative reading frames. J. Exp. Med. 184:1319.
45. Elliott, T., H. Bodmer and A. Townsend, 1996. Recognition of out-of-frame major histocompatibility complex class I-restricted epitopes in vivo. Eur. J. Immunol. 26:1175.
46. Uenaka, A., T. Ono, T. Akisawa, H. Wada, T. Yasuda and E. Nakayama, 1994. Identification of a unique peptide pRL1 on BALB/c RL male 1 leukemia recognized by cytotoxic T lymphocytes and its relation to the aki onogene. J. Exp. Med. 180:1599.
47. Shastri, N., V. Nguyen and F. Gonzalez, 1995. Major histocompatibility class I molecules can present cryptic translation products to T cells. J. Biol. Chem. 270:1088.
48. Malarkannan, S., M. Afkarian and N. Shastri, 1995. A rare cryptic translation product is presented by K^{b} major histocompatibility complex class I molecule to alloreactive T cells. J. Exp. Med. 182:1739.
49. Guilloux, Y., L. Lucas, V.G. Brichard, A. Van Pel, C. Viret, E. De Plaen, F. Brasseur, B. Lethe, F. Jotereau and T. Boon, 1996. A peptide recognized by human cytolytic T lymphocytes on HLA-A2 melanomas is encoded by an intron sequence of the *N*-acetylglucosaminyltransferase V. gene. J. Exp. Med. 183:1173.
50. Robbins, P.F., M. El-Gamil, Y.F. Li, E. Fitzgerald, Y. Kawakami and S.A. Rosenberg, 1997. The intronic region of an incompletely spliced gp100 gene transcript encodes an epitope recognized by melanoma-reactive tumor-infiltrating lymphocytes. J. Immunol. 159:303.

## Claims

1. An isolated cancer peptide comprising the amino acid sequence MLMAQEALAF LMAQGAMLAA QERRVPRAAE VPGAQGQQGP RGREEAPRGV RMAARLQG (SEQ ID NO: 5), or a functional portion or derivative thereof, wherein said functional portion or derivative is immunologically recognized by antigen specific cytotoxic T lymphocytes specific for the peptide having the sequence of SEQ ID NO: 5 of Figure 3A.

2. An isolated cancer peptide, functional portion or derivative, thereof according to claim 1, wherein the cytotoxic T lymphocytes are MHC class I restricted.

3. An isolated cancer peptide, functional portion or derivative thereof, according to claim 1 or 2, wherein the cancer peptide is derived from a cancer selected from the group consisting of: a non-Hodgkins lymphoma, leukemia, Hodgkins lymphoma, lung cancer, liver cancer, metastases, melanoma, adenocarcinoma, thymoma, colon cancer, uterine cancer, breast cancer, prostate cancer, ovarian cancer, cervical cancer, bladder cancer, kidney cancer, pancreatic cancer and sarcoma.

4. An isolated cancer peptide, functional portion or derivative thereof, according to any of claims 1 to 3 wherein the cancer peptide or functional portion or derivative thereof is present on primary breast tumor isolates and melanoma cells.

5. An isolated cancer peptide, portion or derivative thereof, encoded by wherein the portion or derivative thereof is recognized by antigen specific cytotoxic T cells specific for the peptide having the sequence of SEQ ID NO: 5 of Figure 3A.

6. An isolated cancer peptide, portion or derivative thereof, according to claim 5 wherein the peptide comprises the amino acid sequence:
LAAQERRVPR or
AAQERRVPR.

7. A pharmaceutical composition comprising at least one cancer peptide according to any of claims 1 to 6 and a pharmaceutically acceptable carrier.

8. An immunogen comprising the cancer peptide according to any of claims 1 to 6 alone or in combination with at least one immunostimulatory molecule, wherein said immunogen elicits antigen specific cytotoxic T lymphocytes.

9. An immunogen according to claim 8, wherein the immunostimulatory molecule is an HLA molecule.

10. An isolated nucleic acid encoding a cancer peptide according to any of claims 1 to 6, wherein the nucleic acid does not encode SEQ ID NO: 4 of Figure 3A.

11. An isolated nucleic acid according to claim 10, wherein the nucleic acid encodes an alternative open reading frame gene product.

12. The isolated nucleic acid according to claim 11 encoding the amino acid sequence

13. An isolated nucleic acid according to claim 12, wherein the nucleic acid encodes a cancer peptide having the amino acid sequence
LAAQERRVPR or AAQERRVPR.

14. A recombinant expression vector comprising the nucleic acid according to any of claims 10 to 13.

15. An isolated cell transformed or transfected with a recombinant expression vector according to claim 14.

16. An isolated cell according to claim 15, wherein the cell is an antigen presenting cell.

17. An oligonucleotide consisting of a nucleic acid complementary to the portion of the nucleic acid sequence encoding the peptide having the sequence MLMAQEALAF LMAQGAMLAA QERRVPRAAE VPGAQGQQGP RGREEAPRGV RMAARLQG (SEQ ID NO: 5).

18. A recombinant virus comprising a recombinant virus which has incorporated into a viral genome or portion thereof the nucleic acid according to any of claims 10 to 13.

19. A recombinant virus according to claim 18 further comprising at least one gene encoding an immunostimulatory molecule.

20. The recombinant virus according to claim 18, wherein the virus is selected from the group consisting of retrovirus, baculovirus, Ankara virus, fowlpox, adenovirus, and vaccinia virus.

21. The recombinant virus according to claim 18, wherein the nucleic acid encodes a cancer peptide derived from melanocytes.

22. A recombinant virus according to claim 19, wherein the inmounostimulatory molecule is a HLA class I molecule.

23. An isolated cell transformed or transfected with the recombinant virus according to claims 18 to 22.

24. An isolated antibody or antigen binding portion thereof that binds to the cancer peptide encoded by

25. A method of recombinantly producing a cancer peptide, or functional portion or derivative thereof, according to claim 1 comprising:
a. inserting a nucleotide sequence of or portion or variant thereof, into an expression vector;
b. transferring the expression vector into a host cell;
c. culturing the host cell under conditions appropriate for expression of the cancer peptide or functional portion or derivative thereof; and
d. harvesting the recombinant cancer peptide, or functional portion or derviative thereof.

26. A method according to claim 25, further comprising in step (a) inserting a nucleotide sequence encoding an HLA class I molecule, or portion thereof, into the expression vector.

27. An *in vitro* method of detecting the presence of cancer or precancer in a mammal comprising:
a. contacting a nucleic acid complementary to the portion of the nucleic acid sequence of encoding the peptide having the sequence MLMAQEALAF LMAQGAMLAA QERRVPRAA VPGAQGQQGP RGREEAPRGV RMAARLQG (SEQ ID NO:5), with a test biological sample of mRNA taken from the mammal under conditions allowing for a complex to form between the sequence and the mRNA;
b. detecting the complex;
c. comparing the amount of mRNA in the test sample with an amount of mRNA from a known normal biological sample, wherein an increased amount of mRNA from the test sample is indicative of cancer or precancer.

28. A method according to claim 27, wherein the cancer or precancer is melanoma.

29. A method according to claim 27, wherein the biological sample is from breast tissue.

30. The method according to claim 27, wherein the nucleic acid is complementary to the nucleic acid sequence CCTCGGGGCC GGGAGGAGGC GCCCCGCGGG (SEQ ID NO: 51).

31. An *in vitro* method of detecting the cancer peptide, or functional portion or derivative thereof, according to any of claims 1 to 6 in a biological sample comprising:
a. contacting the sample with antibodies specific for said cancer peptide under conditions to form an immune complex, and
b. detecting the presence of the immune complex.

32. Use of an effective amount of the cancer peptide, or functional portion or derivative thereof according to any of claims 1 to 6 alone or in combination with an HLA molecule, for the preparation of a medicament for preventing or inhibiting cancer in a mammal.

33. Use of T lymphocytes for the preparation of a medicament, wherein T lymphocytes are *in vitro* exposed to a cancer peptide, or funtional portion or derivative thereof, according to any of claims 1 to 6 alone or in combination with an MHC molecule, or a fragment thereof, for a time sufficient to elicit cancer peptide specific T lymphocytes.

34. Use of an effective amount of the cancer peptide according to any of claims 1 to 6 alone, or in combination with an HLA molecule or a fragment thereof, said amount is effective in preventing or inhibiting cancer in a mammal, for the preparation of a medicament.

35. Use of an effective amount of a recombinant virus according to any of claims 18 to 22 alone or in combination with an exogenous immunostimulatory molecule, said amount is effective in preventing or inhibiting cancer, for the preparation of a medicament.

36. A pharmaceutical composition comprising the recombinant virus according to any of claims 18 to 22 alone or in combination with an exogenous immunostimulatory molecule, chemotherapy drug, antibiotic, antifungal drug, antiviral drug or combination thereof, and a pharmaceutically acceptable carrier.

37. A non-human transgenic animal carrying and expressing a gene consisting of or portion or derivative thereof,
wherein said portion or derivative encodes a peptide immunologically recognized by antigen specific cytotoxic T lymphocytes specific for the peptide having the sequence of SEQ ID NO: 5 of Figure 3A.

38. An isolated cancer antigen specific human cytotoxic T lymphocyte elicited by the cancer peptide according to any of claims 1 to 6.

39. The cancer antigen specific human cytotoxic T lymphocyte according to claim 38, wherein the lymphocyte recognizes an HLA-A31 molecule.

40. The cancer antigen specific human cytotoxic T lymphocyte according to claim 38, wherein the lymphocyte recognizes an HLA Class I molecule selected from the group consisting of HLA-A3, HLA-A11, HLA-A33, and HLA-A68.

41. Use of T lymphocytes for the preparation of a medicament for inhibiting melanoma in a mammal, wherein T lymphocytes are *in vitro* exposed to a cancer peptide, or portion or variant thereof, according to any of claims 1 to 6 alone or combination with an MHC molecule or a fragment thereof, for a time sufficient to elicit cancer peptide specific T lymphocytes.

42. Use of a recombinant virus according to any of claims 18 to 22, alone or in combination with an immunostimulatory molecule, for the preparation of a medicament for preventing or inhibiting cancer in a mammal.

43. The use according to claim 42, wherein the mammal expresses an HLA Class I molecule from the group consisting of HLA-A31, HLA-A3, HLA-A11, HLA-A33, or HLA-A68.

## Patentansprüche

1. Isoliertes Krebspeptid, das die Aminosäuresequenz MLMAQEALAF LMAQGAMLAA QERRVPRAAE VPGAQGQQGP RGREEAPRGV RMAARLQG (SEQ ID NO: 5) umfasst, oder ein funktionelles Teil oder Derivat davon, wobei das funktionelle Teil oder Derivat von Antigen-spezifischen cytotoxischen T-Lymphozyten immunologisch erkannt wird, die für das Peptid mit der Sequenz von SEQ ID NO: 5 von Figur 3A spezifisch sind.

2. Isoliertes Krebspeptid, funktionelles Teil oder Derivat davon gemäß Anspruch 1, wobei die cytotoxischen T-Lymphozyten MHC-Klasse I-restringiert sind.

3. Isoliertes Krebspeptid, funktionelles Teil oder Derivat davon gemäß Anspruch 1 oder 2, wobei sich das Krebspeptid von einem Krebs ableitet, der ausgewählt ist aus der Gruppe, bestehend aus: einem Nicht-Hodgkin-Lymphom, Leukämie, Hodgkin-Lymphom, Lungenkrebs, Leberkrebs, Metastasen, Melanom, Adenokarzinom, Thymom, Kolonkrebs, Gebärmutterkrebs, Brustkrebs, Prostatakrebs, Ovarialkrebs, Cervixkrebs, Blasenkrebs, Nierenkrebs, Pankreaskrebs und Sarkom.

4. Isoliertes Krebspeptid, funktionelles Teil oder Derivat davon gemäß einem der Ansprüche 1 bis 3, wobei das Krebspeptid oder funktionelle Teil oder Derivat davon auf primären Brusttumorisolaten und Melanomzellen vorhanden ist.

5. Isoliertes Krebspeptid, Teil oder Derivat davon, das von kodiert wird, wobei das Teil oder Derivat davon von Antigen-spezifischen cytotoxischen T-Zellen erkannt wird, die für das Peptid mit der Sequenz von SEQ ID NO: 5 von Figur 3A spezifisch sind.

6. Isoliertes Krebspeptid, Teil oder Derivat davon gemäß Anspruch 5, wobei das Peptid die Aminosäuresequenz:
LAAQERRVPR oder
AAQERRVPR
umfasst.

7. Pharmazeutische Zusammensetzung, die mindestens ein Krebspeptid gemäß einem der Ansprüche 1 bis 6 und einen pharmazeutisch verträglichen Träger umfasst.

8. Immunogen, das das Krebspeptid gemäß einem der Ansprüche 1 bis 6 allein oder zusammen mit mindestens einem immunostimulatorischen Molekül umfasst, wobei das Immunogen Antigen-spezifische cytotoxische T-Lymphozyten hervorruft.

9. Immunogen gemäß Anspruch 8, wobei das immunostimulatorische Molekül ein HLA-Molekül ist.

10. Isolierte Nukleinsäure, die für ein Krebspeptid gemäß einem der Ansprüche 1 bis 6 kodiert, wobei die Nukleinsäure nicht für SEQ ID NO: 4 von Figur 3A kodiert.

11. Isolierte Nukleinsäure gemäß Anspruch 10, wobei die Nukleinsäure für ein Genprodukt eines alternativen offenen Leserahmens kodiert.

12. Isolierte Nukleinsäure gemäß Anspruch 11, die für die Aminosäuresequenz kodiert.

13. Isolierte Nukleinsäure gemäß Anspruch 12, wobei die Nukleinsäure für ein Krebspeptid mit der Aminosäuresequenz
LAAQERRVPR oder AAQERRVPR
kodiert.

14. Rekombinanter Expressionsvektor, der die Nukleinsäure gemäß einem der Ansprüche 10 bis 13 umfasst.

15. Isolierte Zelle, die mit einem rekombinanten Expressionsvektor gemäß Anspruch 14 transformiert oder transfiziert ist.

16. Isolierte Zelle gemäß Anspruch 15, wobei die Zelle eine Antigen-präsentierende Zelle ist.

17. Oligonukleotid, das aus einer Nukleinsäure besteht, die zu dem Teil der Nukleinsäuresequenz das für das Peptid mit der Sequenz kodiert, komplementär ist.

18. Rekombinantes Virus, das ein rekombinantes Virus umfasst, bei dem in ein virales Genom oder ein Teil davon die Nukleinsäure gemäß einem der Ansprüche 10 bis 13 eingebaut ist.

19. Rekombinantes Virus gemäß Anspruch 18, das ferner mindestens ein Gen umfasst, das für ein immunostimulatorisches Molekül kodiert.

20. Rekombinantes Virus gemäß Anspruch 18, wobei das Virus ausgewählt ist aus der Gruppe, bestehend aus Retrovirus, Baculovirus, Ankara-Virus, Geflügelpocken, Adenovirus und Vakziniavirus.

21. Rekombinantes Virus gemäß Anspruch 18, wobei die Nukleinsäure für ein Krebspeptid kodiert, das sich von Melanozyten ableitet.

22. Rekombinantes Virus gemäß Anspruch 19, wobei das immunostimulatorische Molekül ein HLA-Klasse I-Molekül ist.

23. Isolierte Zelle, die mit dem rekombinanten Virus gemäß den Ansprüchen 18 bis 22 transformiert oder transfiziert ist.

24. Isolierter Antikörper oder Antigen-bindendes Teil davon, der/das an das Krebspeptid bindet, das von kodiert wird

25. Verfahren zum rekombinanten Herstellen eines Krebspeptids oder eines funktionellen Teils oder Derivats davon gemäß Anspruch 1, umfassend:
a. Inserieren einer Nukleotidsequenz oder eines Teils oder einer Variante davon in einen Expressionsvektor,
b. Transferieren des Expressionsvektors in eine Wirtszelle,
c. Kultivieren der Wirtszelle unter Bedingungen, die zur Expression des Krebspeptids oder funktionellen Teils oder Derivats davon geeignet sind, und
d. Gewinnen des rekombinanten Krebspeptids oder funktionellen Teils oder Derivats davon.

26. Verfahren gemäß Anspruch 25, das ferner in Schritt (a) ein Inserieren einer Nukleotidsequenz, die für ein HLA-Klasse 1-Molekül kodiert, oder eines Teils davon in den Expressionsvektor umfasst.

27. *In vitro*-Verfahren zum Nachweisen des Vorhandenseins von Krebs oder Vorkrebs in einem Säuger, umfassend:
a. In-Kontakt-Bringen einer Nukleinsäure, die zu dem Teil der Nukleinsäuresequenz das für das Peptid mit der Sequenz
MLMAQEALAF LMAQGAMLAA QERRVPRAAE VPGAQGQQGP RGREEAPRGV RMAARLQG.
(SEQ ID NO:5) kodiert, komplementär ist, mit einer biologischen Testprobe von mRNA, die aus dem Säuger entnommen wurde, unter Bedingungen, die ermöglichen, dass sich ein Komplex zwischen der Sequenz und der mRNA ausbildet,
b. Nachweisen des Komplexes,
c. Vergleichen der Menge an mRNA in der Testprobe mit einer Menge an mRNA aus einer bekannten normalen biologischen Probe, wobei eine erhöhte Menge an mRNA aus der Testprobe für Krebs oder Vorkrebs indikativ ist.

28. Verfahren gemäß Anspruch 27, wobei der Krebs oder Vorkrebs ein Melanom ist.

29. Verfahren gemäß Anspruch 27, wobei die biologische Probe von Brustgewebe abstammt.

30. Verfahren gemäß Anspruch 27, wobei die Nukleinsäure zu der Nukleinsäuresequenz
CCTCGGGGCC GGGAGGAGGC GCCCCGCGGG (SEQ ID NO: 51)
komplementär ist.

31. *In vitro*-Verfahren zum Nachweisen des Krebspeptids oder funktionellen Teils oder Derivats davon gemäß einem der Ansprüche 1 bis 6 in einer biologischen Probe, umfassend:
a. In-Kontakt-Bringen der Probe mit Antikörpern, die für das Krebspeptid spezifisch sind, unter Bedingungen zum Ausbilden eines Immunkomplexes und
b. Nachweisen des Vorhandenseins des Immunkomplexes.

32. Verwendung einer wirksamen Menge des Krebspeptids oder funktionellen Teils oder Derivats davon gemäß einem der Ansprüche 1 bis 6 allein oder zusammen mit einem HLA-Molekül zur Herstellung eines Medikaments zum Verhindern oder Hemmen von Krebs in einem Säuger.

33. Verwendung von T-Lymphozyten zur Herstellung eines Medikaments, wobei die T-Lymphozyten *in vitro* einem Krebspeptid oder funktionellen Teil oder Derivat davon gemäß einem der Ansprüche 1 bis 6 allein oder zusammen mit einem MHC-Molekül oder einem Fragment davon für eine Zeitspanne ausgesetzt werden, die ausreicht, um Krebspeptid-spezifische T-Lymphozyten hervorzurufen.

34. Verwendung einer wirksamen Menge des Krebspeptids gemäß einem der Ansprüche 1 bis 6 allein oder zusammen mit einem HLA-Molekül oder einem Fragment davon, wobei die Menge zum Verhindern oder Hemmen von Krebs in einem Säuger wirksam ist, zur Herstellung eines Medikaments.

35. Verwendung einer wirksamen Menge eines rekombinanten Virus gemäß einem der Ansprüche 18 bis 22 allein oder zusammen mit einem exogenen immunostimulatorischen Molekül, wobei die Menge zum Verhindern oder Hemmen von Krebs wirksam ist, zur Herstellung eines Medikaments.

36. Pharmazeutische Zusammensetzung, die das rekombinante Virus gemäß einem der Ansprüche 18 bis 22 allein oder zusammen mit einem exogenen immunostimulatorischen Molekül, chemotherapeutischen Arzneimittel, Antibiotikum, antifungiziden Arzneimittel, antiviralen Arzneimittel oder einer Kombination davon und einen pharmazeutisch verträglichen Träger umfasst.

37. Nichtmenschliches transgenes Tier, das ein Gen, bestehend aus oder ein Teil oder Derivat davon trägt und exprimiert,
wobei das Teil oder Derivat für ein Peptid kodiert, das durch Antigen-spezifische cytotoxische T-Lymphozyten immunologisch erkannt wird, die für das Peptid mit der Sequenz von SEQ ID NO:5 von Figur 3A spezifisch sind.

38. Isolierter Krebsantigen-spezifischer humaner cytotoxischer T-Lymphozyt, der durch das Krebspeptid gemäß einem der Ansprüche 1 bis 6 hervorgerufen wird.

39. Krebsantigen-spezifischer humaner cytotoxischer T-Lymphozyt gemäß Anspruch 38, wobei der Lymphozyt ein HLA-A31-Molekül erkennt.

40. Krebsantigen-spezifischer humaner cytotoxischer T-Lymphozyt gemäß Anspruch 38, wobei der Lymphozyt ein HLA-Klasse I-Molekül erkennt, das ausgewählt ist aus der Gruppe, bestehend aus HLA-A3, HLA-A11, HLA-A33 und HLA-A68.

41. Verwendung von T-Lymphozyten zur Herstellung eines Medikaments zum Hemmen eines Melanoms in einem Säuger, wobei T-Lymphozyten *in vitro* einem Krebspeptid oder einem Teil oder einer Variante davon gemäß einem der Ansprüche 1 bis 6 allein oder zusammen mit einem MHC-Molekül oder einem Fragment davon für eine Zeitspanne ausgesetzt werden, die ausreicht, um Krebspeptid-spezifische T-Lymphozyten hervorzurufen.

42. Verwendung eines rekombinanten Virus gemäß einem der Ansprüche 18 bis 22 allein oder zusammen mit einem immunostimulatorischen Molekül zur Herstellung eines Medikaments zum Verhindern oder Hemmen von Krebs in einem Säuger.

43. Verwendung gemäß Anspruch 42, wobei der Säuger ein HLA Klasse I-Molekül aus der Gruppe, bestehend aus HLA-A31, HLA-A3, HLA-A11, HLA-A33 oder HLA-A68, exprimiert.

## Revendications

1. Peptide de cancer isolé, comprenant la séquence d'acides aminés MLMAQEALAF LMAQGAMLAA QERRVPRAAE VPGAQGQQGP RGREEAPRGV RMAARLQG (SEQ ID NO: 5), ou une portion fonctionnelle ou un dérivé de celle-ci, tandis que ladite portion fonctionnelle ou ledit dérivé est reconnu immunologiquement par les lymphocytes T cytotoxiques spécifiques pour un antigène, spécifiques pour le peptide ayant la séquence de SEQ ID NO: 5 selon la figure 3A.

2. Peptide de cancer isolé, portion fonctionnelle ou dérivé de celui-ci selon la revendication 1, dans lequel les lymphocytes T cytotoxiques sont restreints à la classe I du CMH.

3. Peptide de cancer isolé, portion fonctionnelle ou dérivé de celui-ci, selon la revendication 1 ou 2, dans lequel le peptide de cancer est dérivé d'un cancer choisi dans le groupe consistant en: lymphome non-hodgkinien, leucémie, lymphome de Hodgkin, cancer du poumon, cancer du foie, métastases, mélanome, adénocarcinome, thymome, cancer du côlon, cancer de l'utérus, cancer du sein, cancer de la prostate, cancer des ovaires, cancer cervical, cancer de la vessie, cancer du rein, cancer du pancréas et sarcome.

4. Peptide de cancer isolé, portion fonctionnelle ou dérivé de celui-ci, selon l'une quelconque des revendications 1 à 3, dans lequel le peptide de cancer ou la portion fonctionnelle ou le dérivé de celui-ci est présent sur des isolats de tumeur du sein primaire et sur les cellules de mélanome.

5. Peptide de cancer isolé, portion ou dérivé de celui-ci, codé par tandis que la portion ou le dérivé de celui-ci est reconnu par les cellules T cytotoxiques spécifiques pour un antigène, spécifiques pour le peptide ayant la séquence SEQ ID NO: 5 selon la Figure 3A.

6. Peptide de cancer isolé, portion ou dérivé de celui-ci, selon la revendication 5, dans lequel le peptide comprend la séquence d'acides aminés:
LAAQERRVPR ou AAQERRVPR.

7. Composition pharmaceutique comprenant au moins un peptide de cancer selon l'une quelconque des revendications 1 à 6 et un support pharmaceutiquement acceptable.

8. Agent immunogène comprenant le peptide de cancer selon l'une quelconque des revendications 1 à 6, seul ou en combinaison avec au moins une molécule immunostimulatrice, tandis que ledit agent immunogène provoque les lymphocytes T cytotoxiques spécifiques pour un antigène.

9. Agent immunogène selon la revendication 8, dans lequel la molécule immunostimulatrice est une molécule de HLA.

10. Acide nucléique isolé codant pour un peptide de cancer selon l'une quelconque des revendications 1 à 6, dans lequel l'acide nucléique ne code pas pour SEQ ID NO: 4 selon la Figure 3A.

11. Acide nucléique isolé selon la revendication 10, dans lequel l'acide nucléique code pour un produit génique du cadre de lecture ouvert alternatif.

12. Acide nucléique isolé selon la revendication 11, codant pour la séquence d'acides aminés MLMAQEALAF LMAQGAMLAA QERRVPRAAE VPGAQGQQGP RGREEAPRGV RMAARLQG.

13. Acide nucléique isolé selon la revendication 12, dans lequel l'acide nucléique code pour un peptide de cancer ayant la séquence d'acides aminés:
LAAQERRVPR ou AAQERRVPR.

14. Vecteur d'expression recombinant comprenant l'acide nucléique selon l'une quelconque des revendications 10 à 13.

15. Cellule isolée transformée ou transfectée avec un vecteur d'expression recombinant selon la revendication 14.

16. Cellule isolée selon la revendication 15, dans laquelle la cellule est une cellule présentant l'antigène.

17. Oligonucléotide consistant en un acide nucléique complémentaire de la portion de la séquence d'acide nucléique codant pour le peptide ayant la séquence MLMAQEALAF LMAQGAMLAA QERRVPRAAE VPGAQGQQGP RGREEAPRGV RMAARLQG (SEQ ID NO:5).

18. Virus recombinant comprenant un virus recombinant qui a incorporé dans un génome viral ou une portion de celui-ci l'acide nucléique selon l'une quelconque des revendications 10 à 13.

19. Virus recombinant selon la revendication 18, comprenant en outre au moins un gène codant pour une molécule immunostimulatrice.

20. Virus recombinant selon la revendication 18, dans lequel le virus est choisi dans le groupe consistant en rétrovirus, baculovirus, virus d'Ankara, virus du molluscum contagiosum, adénovirus, et virus de la vaccine.

21. Virus recombinant selon la revendication 18, dans lequel l'acide nucléique code pour un peptide de cancer dérivé de mélanocytes.

22. Virus recombinant selon la revendication 19, dans lequel la molécule immunostimulatrice est une molécule de HLA de classe I.

23. Cellule isolée transformée ou transfectée avec le virus recombinant selon les revendications 18 à 22.

24. Anticorps isolé ou portion de liaison à l'antigène de celui-ci qui se lie au peptide de cancer codé par

25. Procédé pour la production par voie recombinante d'un peptide de cancer, ou d'une portion fonctionnelle ou d'un dérivé de celui-ci, selon la revendication 1, comprenant:
a. l'insertion d'une séquence nucléotidique de ou d'une portion ou variante de celle-ci, dans un vecteur d'expression;
b. le transfert du vecteur d'expression dans une cellule hôte;
c. la culture de la cellule hôte dans des conditions appropriées pour l'expression du peptide de cancer ou d'une portion fonctionnelle ou d'un dérivé de celui-ci; et
d. la récolte du peptide de cancer recombinant, ou d'une portion fonctionnelle ou d'un dérivé de celui-ci.

26. Procédé selon la revendication 25, comprenant en outre dans l'étape (a) l'insertion d'une séquence nucléotidique codant pour une molécule de HLA de classe I, ou d'une portion de celle-ci, dans le vecteur d'expression.

27. Procédé *in vitro* pour la détection de la présence d'un cancer ou d'un précancer chez un mammifère, comprenant:
a. la mise en contact d'un acide nucléique complémentaire de la portion de la séquence d'acide nucléique de codant pour le peptide ayant la séquence MLMAQEALAF LMAQGAMLAA QERRVPRAAE VPGAQGQQGP RGREEAPRGV RMAARLQG (SEQ ID NO: 5), avec un échantillon biologique d'ARNm d'essai prélevé sur le mammifère dans des conditions permettant à un complexe de se former entre la séquence et l'ARNm;
b. la détection du complexe;
c. la comparaison de la quantité d'ARNm dans l'échantillon d'essai avec une quantité d'ARNm provenant d'un échantillon biologique normal connu, tandis qu'une quantité accrue de l'ARNm provenant de l'échantillon d'essai est un indice de cancer ou de précancer.

28. Procédé selon la revendication 27, dans lequel le cancer ou le précancer est un mélanome.

29. Procédé selon la revendication 27, dans lequel l'échantillon biologique provient d'un tissu du sein.

30. Procédé selon la revendication 27, dans lequel l'acide nucléique est complémentaire de la séquence d'acide nucléique CCTCGGGGCC GGGAGGAGGC GCCCCGCGGG (SEQ ID NO: 51).

31. Procédé *in vitro* pour la détection du peptide de cancer, ou d'une portion fonctionnelle ou d'un dérivé de celui-ci, selon l'une quelconque des revendications 1 à 6 dans un échantillon biologique, comprenant:
a. la mise en contact de l'échantillon avec des anticorps spécifiques pour ledit peptide de cancer dans des conditions aptes à former un complexe immun, et
b. la détection de la présence du complexe immun.

32. Utilisation d'une quantité efficace du peptide de cancer, ou d'une portion fonctionnelle ou d'un dérivé de celui-ci selon l'une quelconque des revendications 1 à 6, seul ou en combinaison avec une molécule de HLA, pour la préparation d'un médicament pour la prévention ou l'inhibition d'un cancer chez un mammifère.

33. Utilisation de lymphocytes T pour la préparation d'un médicament, dans laquelle des lymphocytes T sont exposés *in vitro* à un peptide de cancer, ou à une portion fonctionnelle ou à un dérivé de celui-ci selon l'une quelconque des revendications 1 à 6, seul ou en combinaison avec une molécule de CMH, ou un fragment de celle-ci pendant une durée suffisante pour provoquer des lymphocytes T spécifiques pour le peptide du cancer.

34. Utilisation d'une quantité efficace du peptide de cancer selon l'une quelconque des revendications 1 à 6, seul ou en combinaison avec une molécule de HLA ou un fragment de celle-ci, ladite quantité étant efficace dans la prévention ou l'inhibition d'un cancer chez un mammifère, pour la préparation d'un médicament.

35. Utilisation d'une quantité efficace d'un virus recombinant selon l'une quelconque des revendications 18 à 22, seul ou en combinaison avec une molécule immunostimulatrice exogène, ladite quantité étant efficace dans la prévention ou l'inhibition d'un cancer, pour la préparation d'un médicament.

36. Composition pharmaceutique comprenant le virus recombinant selon l'une quelconque des revendications 18 à 22, seul ou en combinaison avec une molécule immunostimulatrice exogène, un médicament pour chimiothérapie, un antibiotique, un médicament antifongique, un médicament antiviral ou une combinaison de ceux-ci, et un support pharmaceutiquement acceptable.

37. Animal transgénique non-humain portant et exprimant un gène consistant en ou une portion ou un dérivé de celle-ci,
tandis que ladite portion ou ledit dérivé code pour un peptide reconnu immunologiquement par des lymphocytes T cytotoxiques spécifiques de l'antigène, spécifiques pour le peptide ayant la séquence de SEQ ID NO: 5 selon la Figure 3A.

38. Lymphocyte T cytotoxique humain spécifique d'un antigène de cancer isolé, provoqué par le peptide de cancer selon l'une quelconque des revendications 1 à 6.

39. Lymphocyte T cytotoxique humain spécifique d'un antigène de cancer selon la revendication 38, dans lequel le lymphocyte reconnaît une molécule de HLA-A31.

40. Lymphocyte T cytotoxique humain spécifique d'un antigène de cancer selon la revendication 38, dans lequel le lymphocyte reconnaît une molécule de HLA de classe I choisie dans le groupe consistant en HLA-A3, HLA-A11, HLA-A33, et HLA-A68.

41. Utilisation de lymphocytes T pour la préparation d'un médicament pour l'inhibition d'un mélanome chez un mammifère, dans laquelle des lymphocytes T sont exposés *in vitro* à un peptide de cancer, ou à une portion ou une variante de celui-ci, selon l'une quelconque des revendications 1 à 6, seul ou en combinaison avec une molécule de CMH ou un fragment de celle-ci, pendant une durée suffisante pour provoquer les lymphocytes T spécifiques du peptide de cancer.

42. Utilisation d'un virus recombinant selon l'une quelconque des revendications 18 à 22, seul ou en combinaison avec une molécule immunostimulatrice, pour la préparation d'un médicament pour la prévention ou l'inhibition d'un cancer chez un mammifère.

43. Utilisation selon la revendication 42, dans laquelle le mammifère exprime une molécule de HLA de classe I appartenant au groupe consistant en HLA-A31, HLA-A3, HLA-A11, HLA-A33, ou HLA-A68.
